# EUROPEAN PATENT APPLICATION

(11) **EP 4 129 328 A1**
(43) Date of publication of application: **08.02.2023**
(21) Application number: 21306095.7
(22) Date of filing: 05.08.2021
(51) Int. Cl.: A61K 39/002, C12N 15/63

(54) **GENETICALLY MODIFIED PLASMODIUM PARASITE EXPRESSING IL-6 - USE TO RAISE AN IMMUNE RESPONSE IN A HOST AGAINST MALARIA AT THE PRE-ERYTHROCYTIC STAGE OF DEVELOPMENT OF THE PARASITE**

(71) Applicant: INSTITUT PASTEUR, 75724 Paris Cedex 15 (FR)
(72) Inventor: MECHERI, Salah, 75724 PARIS CEDEX 15 (FR); PERONET, Roger, 75724 PARIS CEDEX 15 (FR)
(74) Representative: Ernest Gutmann - Yves Plasseraud S.A.S.

(57) **Abstract**

The present invention relates to recombinant Plasmodium parasites expressing IL-6 protein and their application in raising an immune response in a mammalian host against *Plasmodium* infection or against malaria disease, in a particular a protective response against early forms of parasite development.

## Description

### Field of the invention

The present invention relates to recombinant *Plasmodium* parasites expressing IL-6 protein and their application in raising an immune response in a host against a *Plasmodium* infection or against malaria disease, in a particular in eliciting a protective response against early forms of parasite development in the infected host.

### Background of the invention

Malaria is an infectious disease caused by a eukaryotic single-cell parasite of the *Plasmodium genus.* Malaria control programs have been designed that mainly rely on the use of insecticides and antiplasmodial medicines The occurrence of the resistance of *Anopheles* mosquitoes to insecticides and of the growing chemoresistance of *P. falciparum* to antimalarial drugs make the situation even more severe to elaborate interventions emphasizing the interest of developing an efficient malaria vaccine as an additional tool against the disease burden. Despite many attempts since the 1970s, no vaccine is yet licensed, highlighting the challenges, including the complexity of the development of the parasite, mechanisms for evading the host immune system, and antigenic variation of the parasite. In recent years, in addition to the development of subunit vaccines and radiation-attenuated sporozoites (RAS), researchers have used rodent models to test the efficacy of genetically attenuated parasites (GAPs) as vaccines against pre-erythrocytic and blood-stage malaria infections. Most studies have focused on pre-erythrocytic GAPs, which include parasite mutants blocked early during their development in the liver, such as those lacking integrity of the parasitophorous vacuole, or late during development, such as those with a deficient fatty acid biosynthesis type II pathway (Nganou-Makamdop and Sauerwein, 2013). Some of these pre-erythrocytic GAPs have been tested in humans, e.g., the early-blocked *P. falciparum* Δp52Δp36Δsap1 (Kublin et al., 2017). Likewise, earlier studies showed that *P. berghei* parasites deficient in apicoplast protein important for liver merozoite formation (PALM) displayed a strong impairment in liver merozoite formation with a severe delay in blood-stage infection and a reduced incidence of ECM (Haussig et al., 2011).

Few molecules of malaria parasites have been shown to counteract host innate immunity. In early liver stages, the major sporozoite surface protein called circumsporozoite protein translocates into the hepatocyte cytosol and nucleus, where it outcompetes NF-kB nuclear import and suppresses hundreds of genes involved in the host inflammatory response. It was reported that several parasites including *Plasmodium, Brugia, Trichuris, Eimeiria, Trichinella,* and *Onchorcerca,* all express the migration inhibitory factor (MIF) orthologue of mammalian MIF with significant homology to human MIF. MIF was shown to increase inflammatory cytokine production during the blood phase of *Plasmodium* infection in rodents and to induce antigen-specific CD4 T-cells to develop into short-lived effector cells rather than into memory cells, causing decreased CD4 T-cell recall responses to homologous parasites (Sun et al., 2012). However, parasites lacking MIF were shown to have no growth defect throughout the parasite life cycle in *P. berghei* (Augustijn et al., 2007) or a growth defect during liver-stage development in *P. yoelii* (Miller et al., 2012). The inventors have investigated the protection mediated by an erythrocytic GAP depleted of the gene encoding the immunomodulatory and secreted molecule histamine releasing factor (HRF), using the parasite strain *P. berghei* NK65 that does not cause cerebral malaria and rapid death. In earlier work, they found that blood-stage infection by the mutant self-resolved at day 12 p.i., displaying an immune signature that comprised elevated IL-6 levels, activation of T and B cells, and antigen-specific IgG2c production (Demarta-Gatsi et al., 2016).

Most of the attenuated malaria parasites and other pathogenic microorganisms were obtained by gene deletion whereas recombinant *Plasmodium* parasites expressing selected genes that have functions other than attenuating parasite pathogenicity are very scarce. One typical example was the introduction of the murine IFN-γ into the *Leishmania* parasite with the capacity to secrete the active cytokine into the media. More interestingly, infection with IFN-γ-containing parasites resulted in significantly less severe disease as compared to infection with parasites containing the empty plasmid (Tobin et al., 1993). Transfected *Trypanosoma cruzi* parasites with the murine CD40L gene, which was shown to be transcribed, and translated into protein, when inoculated into mice, a very low level of parasitemia and no mortality were seen with the transfected strain compared to the wild-type strain. Moreover, mice surviving infection with transfected parasites resisted a challenge infection with the wild-type strain (Chamekh et al., 2005). Likewise, transgenic *Leishmania major* parasites expressing CD40L mRNA and protein were developed and mice infected with these parasites developed significantly smaller lesions containing fewer parasites than animals infected with wild-type organisms (Field et al., 2007). Taken together, these data demonstrate the feasibility of generating parasite strains expressing a bioactive host costimulatory molecule that may counteract the poor immunogenicity induced by the parasite during infection and enhances protective immunity against a challenge infection. Other engineered recombinant *Leishmania major* strains to express biologically active granulocyte-macrophage colony-stimulating factor (GM-CSF) were found to survive poorly in macrophages *in vitro* and produce delayed, but not suppressed, lesion development in susceptible mice *in vivo* (Dumas et al., 2003). However, in these reports the authors have not investigated, in pathogen challenge experiments, the immunological protection mechanisms nor did they explore the duration of the immune memory response if it existed. Neither do these experiments relate to parasites having a life cycle of the type of the life cycle of the malaria parasites, especially encompassing distinct but interrelated stages of parasite development in the host and besides dealing with specific adaptation of parasite by tolerance mechanisms.

During the last decade, in search of key mechanisms that establish how the host inflammatory response impacts malaria pathogenesis, the present inventors discovered a set of host as well as parasite genes that turned out to be critical for disease severity. Indeed, approaching the parasite infection in mice from different angles, and using either mutant parasites (i.e *Pb*HRFΔ (Mathieu et al., 2015); (Demarta-Gatsi et al., 2017; Demarta-Gatsi et al., 2016) or genetically altered hosts (i.e NMR2^{-/-}) (Grand et al., 2020), it was striking that common signaling pathways, namely IL-6 production, was constantly found as a key mechanism that not only blocks parasite development, but more importantly allows the initiation of a robust and long-lasting anti-parasite immunity. This is probably an evolutionary conserved mechanism that evolved to eradicate the infection and against which the parasite has selected genes, such as *HRF,* making it more elusive with regard to the host immune system and creating a liver tolerance that should be overcome by an efficient vaccine. IL-6 is known to be a key cytokine that supports the growth and enhancement of antibody production by B cells (Hirano et al., 1986), and in support of this, IL-6-deficient mice are impaired in their IgG production upon immunization (Kopf et al., 1994). On the other hand, IL-6 is critical in regulating CD4 T cell differentiation by promoting IL-4 production during T cell activation and IL-21 production, an essential effector cytokine produced by Tfh cells (Barash et al., 2010) (Hirano et al., 1986). Moreover, IL-6 in combination with IL-7 signaling promotes CD8 memory T cell generation after vaccination (Castellino and Germain, 2007).

### Summary of the invention

As part of their interest in developing an effective live-vaccine strategy, the inventors postulated that transgenic *Plasmodium* parasites expressing IL-6 cytokine would not only induce less pathology but also may provide protection against wild-type challenge with the parasite. As a proof of concept recombinant *Plasmodium berghei* parasites were engineered which express and secrete high levels of biologically active IL-6 and their potential to impair the course of the disease or to prevent its onset or development and accordingly to confer protection against wild-type parasite challenge was evaluated in a suitable animal model that was known to be sensible to infection by *Plasmodium berghei.*

Interestingly, the inventors have now shown that IL-6-mediated abortive parasite development following the transgenic parasite inoculation initiates the priming of the immune system and the acquisition of a long-lasting protection. Considering IL-6 as a critical pro-inflammatory signal from the host in response to the parasite infection, the inventors decided to design and engineer a construct whereby the parasite itself encodes for the IL-6 protein. They coined this approach as an IL-6 suicide gene strategy that may lead to an original vaccine design that would be the first in its kind.

The invention accordingly relates to a transgene construct which comprises a polynucleotide encoding a mammalian IL-6 protein, in particular a human IL-6 protein, wherein the polynucleotide is under the control of a transcription and expression control nucleic acid comprising a promoter of a gene of a *Plasmodium* parasite wherein this gene is selected among genes expressed during exoerythrocytic stage of *Plasmodium* parasite life cycle in a mammalian host, and wherein the transgene comprises a nucleic acid encoding a signal peptide (secretory signal peptide) enabling secretion of the expressed IL-6 protein.

In a particular embodiment, the transgene construct comprises the following nucleic acid sequences from 5' to 3' in the polynucleotide:
- a nucleotide sequence of the promoter of a gene of a *Plasmodium* parasite,
- a nucleotide sequence encoding a signal peptide,
- a nucleotide sequence encoding the IL-6 protein,
- optionally sequence(s) of restriction site(s) suitable for cloning of the above sequences in the transgene and/or for insertion of the transgene construct in the *Plasmodium* parasite genome,
wherein the nucleic acid sequences are functionally associated within the transgene to enable expression of the IL-6 protein from a *Plasmodium* parasite genetically modified with the transgene.

In a particular embodiment, the nucleotide sequence encoding a signal peptide encodes a signal peptide of a gene of a *Plasmodium* parasite.

In a particular embodiment, the nucleotide sequence encoding the IL-6 protein encodes a mammalian IL--, especially the murine or the human IL-6.

In a particular embodiment of the transgene construct, the transcription and expression control nucleic acid comprises a promoter of a *Plasmodium* gene which is expressed in the hepatic stage (also named liver stage) of the *Plasmodium* parasite life cycle, such as a gene expressed in exoerythrocytic forms (EEF ), in particular in late EEFs of the parasite.

In a particular embodiment of the transgene construct, the polynucleotide encoding the mammalian IL-6 protein comprises a nucleotide sequence encompassing the Open Reading Frame (ORF) of the IL-6 gene and is selected from the group of : SEQ ID NO. 1, SEQ ID NO. 2, SEQ ID NO. 3, SEQ ID NO. 4, wherein SEQ ID NO. 1 and SEQ ID NO. 2 each encode the murine IL-6 and SEQ ID NO.3 and SEQ ID NO. 4 each encode the human IL-6 .

In a particular embodiment of the transgene construct, the promoter of the gene of a *Plasmodium* parasite is the promoter of a gene selected from the group of the LISP-2 gene of P. *Berghei* (SEQ ID No. 10), LISP-2 gene of P *falciparum* (SEQ ID No. 11) and LISP-1gene of P. *falciparum* (SEQ ID No. 12).

In a particular embodiment of the transgene construct, the promoter of the gene of a *Plasmodium* parasite is the promoter of a gene selected from the group of the LISP-2 gene of P. *Berghei* (SEQ ID No. 10), the LISP-1 gene of P. *Berghei*, when the ORF encodes murine IL-6, and LISP-2 gene of P *falciparum* (SEQ ID No. 11), LISP2 gene of *P.vivax,* when the ORF encodes human IL-6 preferably the promoter is from the LISP-2 gene of P. *falciparum.*

In a particular embodiment of the transgene construct, the promoter of the gene of a *Plasmodium* parasite is the promoter of the *uis 3* gene of P. *Berghei* when the ORF encodes murine IL-6 or LISP-1gene of P. *falciparum* (SEQ ID No. 12), P. *vivax,* P. *malariae* or P. *ovale* or P. *knowlesi* when the ORF encodes human IL-6, preferably the promoter is from the LISP-1 gene of P. *falciparum.*

In a particular embodiment of the transgene construct, the nucleic acid encoding a signal peptide enabling secretion of the expressed IL-6 protein is selected from the group of : the nucleic acid sequence encoding the signal peptide of *P. berghei* LISP2 gene and having the sequence of SEQ ID No. 5 , the nucleic acid sequence encoding the signal peptide of *P. berghei* LISP1 gene, the nucleic acid sequence of SEQ ID No. 6 encoding the signal peptide of SEQ ID No.7 (MWWRLWWLLLLLLLLWPMVWA) identified according to the Hidden Markov Model (HMM) (this signal peptide has been validated in various systems as published in Barash S. et al 2002), and the nucleic acid sequence of SEQ ID No. 8 encoding the signal peptide of the human albumin gene having the sequence of SEQ ID No.9. (MKWVTFISLLFLFSSAYS)

In a particular embodiment of the transgene construct, the polynucleotide comprises from 5' to 3':
a. a nucleotide sequence of the promoter of a gene of a *Plasmodium* parasite, selected from the group of SEQ ID NO. 10, SEQ ID NO. 11 (both promoters of the LISP2 gene) and SEQ ID NO. 12 (promoter of PfLISP1 gene),
b. a nucleotide sequence encoding a signal peptide, in particular a nucleotide sequence of SEQ ID NO. 5 encoding a signal peptide of a gene of a *Plasmodium* parasite, a nucleotide sequence of SEQ ID NO. 6, or a nucleotide sequence of SEQ ID NO. 8,
c. a nucleotide sequence encoding the IL-6 protein, selected from the group of SEQ ID NO. 1, SEQ ID NO. 2, SEQ ID NO. 3 and SEQ ID No. 4,
d. optionally nucleotide sequence(s) of restriction site(s) suitable for cloning of sequences a., b. and c., in the transgene and/or for insertion of the transgene construct in the *Plasmodium* parasite genome.

In a particular embodiment, the transgene construct comprises the nucleic acid of SEQ ID NO. 15.

The invention also concerns an expression plasmid vector recombined with the transgene construct as disclosed herein.

In a further aspect, the invention relates to a transgenic *Plasmodium* parasite wherein the transgene encodes a mammalian IL-6 protein, in particular a human IL-6 protein and is a transgene construct as disclosed herein.

In a particular embodiment of the transgenic *Plasmodium* parasite, the transgene comprises a nucleotide sequence selected from the group of : SEQ ID NO. 1, SEQ ID NO. 2, SEQ ID NO. 3 and SEQ ID NO. 4. In a particular embodiment, the transgene comprises the nucleic acid of SEQ ID NO. 15.

In a particular embodiment of the transgenic *Plasmodium* parasite, the transgene is integrated in the *Plasmodium* genome.

In a particular embodiment, the transgenic *Plasmodium* parasite is a live attenuated *Plasmodium* parasite capable of infecting human. In a particular embodiment, it is a genetically attenuated parasite (GAP). In a particular embodiment the live attenuated *Plasmodium* parasite originates from *P. falciparum.*

In a particular embodiment, the live attenuated transgenic *Plasmodium* parasite is a preerythrocytic live attenuated *Plasmodium* parasite capable of infecting human, in particular a preerythrocytic genetically attenuated parasite (GAP). In a particular embodiment the preerythrocytic live attenuated *Plasmodium* parasite originates from *P. falciparum.*

In a particular embodiment, the transgenic *Plasmodium* parasite is a transgenic parasite of *Plasmodium falciparum, Plasmodium vivax, Plasmodium malariae, Plasmodium. knowlesi* or *Plasmodium ovale,* in particular a live attenuated transgenic parasite especially a preerythrocytic live attenuated *Plasmodium* parasite as disclosed herein.

In a particular embodiment, the transgenic *Plasmodium* parasite is a sporozoite, selected from the group of infectious sporozoite, genetically attenuated sporozoite or radiation attenuated sporozoite. In a particular embodiment, the sporozoite originate from *Plasmodium Berghei* or from *Plasmodium falciparum.*

In a particular embodiment, the transgenic *Plasmodium* parasite is more specifically an exoerythrocytic form (EEF) of the parasite, in particular an early EEF.

The invention also relates to a composition suitable for inducing, promoting or enhancing resistance against *Plasmodium* parasite infection in a mammalian host wherein the composition comprises a transgenic *Plasmodium* parasite capable of infecting a human host, in particular a transgenic parasite of *Plasmodium falciparum, Plasmodium vivax, Plasmodium malariae, Plasmodium. knowlesi* or *Plasmodium ovale* species, preferably *Plasmodium falciparum,* wherein the transgene is any of the constructs as disclosed herein.

In a particular embodiment, the composition is devoid of an exogenous adjuvant of the immune response or of an immunomodulator.

In a particular embodiment, the composition comprises sporozoites of a transgenic *Plasmodium berghei.*

In a particular embodiment, the composition comprises sporozoites of a transgenic *Plasmodium* parasite capable of infecting a human host, in particular of *Plasmodium falciparum, Plasmodium vivax, Plasmodium malariae, Plasmodium. knowlesi* or *Plasmodium ovale, especially of Plasmodium falciparum* parasite. The amount of sporozoites is determined for administration of the composition to a mammalian host, in particular a human host.

In a particular embodiment, the composition is formulated for *in vivo* transfection of the transgenic parasite in a mammalian host, in particular a human host.

In another aspect the invention relates to methods and uses of the transgenic *Plasmodium* parasites in particular of *Plasmodium falciparum, Plasmodium vivax, Plasmodium malariae, Plasmodium. knowlesi* or *Plasmodium ovale, especially of Plasmodium falciparum* parasite, for the elicitation of an immune response against *Plasmodium* infection in a host or against malaria disease. In a particular embodiment, the administered transgenic *Plasmodium* parasite primes a specific immune response against a *Plasmodium* parasite.

Accordingly, the transgenic *Plasmodium* parasite or the composition as disclosed herein is for use in transfecting cells in a mammalian host, in particular in a human host, susceptible to infection by a *Plasmodium* parasite of the same species, for preventing infection by such *Plasmodium* parasite, for alleviating the outcome of infection, or for preventing malaria disease following infection by such *Plasmodium* parasite, each of these enabling or promoting resistance against *Plasmodium* parasite infection. In an advantageous embodiment, the transgenic *Plasmodium* parasite or the composition enables prevents infection by a *Plasmodium* parasite of a different species or for preventing malaria disease following infection by such *Plasmodium* parasite of a different species. In a particular embodiment, the transgenic *Plasmodium* parasite or the composition as disclosed herein is for use in a mammalian host, in particular in a human host, susceptible to infection by a *Plasmodium* parasite of the same species, for preventing the growth of the *Plasmodium* parasite toward the erythrocytic stage of the parasite life cycle in the host or the release of the parasite from the host liver.

In a particular embodiment, the transgenic *Plasmodium* parasite or the composition as disclosed herein is for use in a mammalian host, in particular in a human host, susceptible to infection by a *Plasmodium* parasite of the same species, for eliciting an immune response in the host wherein the immune response comprises a cellular CD8+ T cell immune response. The immune response may also comprise elicitation of an antibody response.

In an embodiment such transgenic *Plasmodium* parasite or composition is administered to the mammalian host, in particular human host, in conditions enabling priming or eliciting of an immune response by the host against *Plasmodium* infection or for preventing the onset or the development of malaria disease.

In a particular embodiment, the transgenic *Plasmodium* parasite or the composition is for use in eliciting, e.g. priming and/or boosting the innate immune and/or the adaptive immune response against *Plasmodium* liver-stage infection or against *Plasmodium* development in a mammalian host, in particular a human host or against malaria disease.

In a particular embodiment, the transgenic *Plasmodium* parasite or the composition is for use in preventive protection against malaria disease in a mammalian, especially human, host, in particular by conferring sterile protection to the mammalian host, in particular human host.

In a particular embodiment the transgenic *Plasmodium* parasite or the composition is for use in an administration regimen wherein the dose(s) of administered transgenic parasite is from 10⁵ to 10⁶ sporozoites of the transgenic parasite, in particular 1. 35x10⁵ to 9.0x10⁵ sporozoites of the transgenic parasite based on data known in the art (Roestenberg M. et al. 2020), in a human host.

In a particular embodiment, the transgenic *Plasmodium* parasite or the composition is for use in an administration regimen that comprises administration of at least two doses of the transgenic *Plasmodium* parasite or of the composition and/or comprises a prime-boost regimen. In a particular embodiment, two or three doses of the transgenic *Plasmodium* parasite or the composition are administered to the host.

The invention also concerns a method for *in vitro* preparing a transgenic *Plasmodium* parasite according to the invention, wherein the method comprises:
- Providing a *Plasmodium* parasite and transforming this *Plasmodium* parasite with a transgenic construct of the invention encoding the IL-6 protein, to enable integration of this construct in the *Plasmodium* parasite, in particular to enable stable integration of this construct in the *Plasmodium* parasite,
- Recovering the transgenic *Plasmodium* parasite recombined with the transgene construct expressing the IL-6 protein as a biologically active protein.

In a particular embodiment, the *Plasmodium* parasite is transfected with a plasmid vector comprising the transgenic construct of the invention that encodes the IL-6 protein. The transfection step may be carried out by nucleofection according to methods known in the art (Janse et al 2006).

### Detailed description of the invention

According to the invention, a **"promoter of a gene of a *Plasmodium* parasite"** is a promoter of an endogenous gene of a *Plasmodium* parasite, i.e. is a promoter that enables expression of a gene originating from (e.g. naturally arising within) a wild-type *Plasmodium* parasite or from a variant thereof (especially a mutant) that is infectious for a mammalian host, in particular a human host.

When used in a transgene construct of the invention such promoter advantageously enables expression of the IL-6 nucleotide sequence of the construct in a manner similar as would be expressed the *Plasmodium* endogenous gene naturally controlled by the promoter.

The endogenous gene suitable for providing the promoter controlling the expression of the IL-6 protein may be advantageously selected among genes that are specifically expressed during the pre-erythrocytic stage of the parasite life cycle in the host, especially during the liver-stage of the parasite life-cycle.

Among promoters suitable for the expression of the IL-6 protein in a transgenic *Plasmodium* parasite, the following examples are cited:
- the promoter (SEQ ID No 11) of the LISP2 gene of P. *berghei.*
   **LISP2** protein (Plasmodium liver-specific protein 2 or LISP-2) is 2038 amino-acids (aa) and 2519 aa long in *P. cynomolgi* and *P. vivax,* with an N-terminal signal peptide and a conserved C-terminal 6-cysteine domain (6-cys) found in a family of apicomplexan-specific proteins which are mostly surface proteins (Arredondo and Kappe 2017 International Journal for Parasitology. 2017;47:409-423. doi: 10.1016/j.ijpara.2016.10.002. ; Orito et al, 2013. Molecular Microbiology. 2013;87:66-79. doi: 10.1111/mmi.12083.). The LISP2 gene may also be originating from P. *falciparum* and its promoter suitable to carry out the invention has the sequence of SEQ ID No 11. It is expressed as a liver-stage specific protein of the parasite.
- the promoter (SEQ ID No 12) of the LISP1 gene of P. *falciparum.*
   **LISP1** protein (Plasmodium liver-specific protein 1 or LISP-1) is a protein expressed in the liver-stage of the life cycle of the parasite in *Plasmodium falciparum* and also in *P. vivax, P. malariae, P. ovale* or *P. knowlesi.*

The **"IL-6 gene"** or "IL-6 sequence" or the "IL-6 ORF" for use according to the invention refers to a nucleic acid molecule comprising or consisting in the genetic information encoding the IL-6 (interleukin-6) protein. In a particular embodiment, the IL-6 protein is the human IL-6 protein (SEQ ID No. 3) and the IL-6 ORF is the Open Reading Frame of the human gene. The nucleic acid molecule may hence be the molecule originating from the wild-type human IL-6 gene and encompasses accordingly the sequence of SEQ ID No.3. Alternatively it may be a nucleotide sequence having at least 70% identity, more preferably at least 80% identity ; more preferably at least 90% identity, more preferably at least 91% identity, more preferably at least 92% identity, more preferably at least 93% identity, more preferably at least 94% identity, more preferably at least 95% identity, more preferably at least 96% identity, more preferably at least 97% identity, more preferably at least 98% identity, or more preferably at least 99% identity with one of the IL-6 coding sequences disclosed herein. In a particular embodiment, the IL-6 ORF is an optimized sequence, especially a codon-optimized sequence of one of the herein disclosed sequences, in particular a codon-optimized sequence with respect to the wild-type sequence of the IL-6 ORF, wherein codon-optimization is performed to enable or improve expression in the *Plasmodium* parasite, in particular in P. *berghei* of in P. *falciparum.* Such codon-optimized sequence encoding human IL-6 is illustrated by the sequence of SEQ ID No.4. In another embodiment the nucleic acid molecule may be the molecule originating from the wild-type murine IL-6 gene and encompasses accordingly the sequence of SEQ ID No.1. Alternatively it may be a codon-optimized sequence wherein codon-optimization is performed to enable expression in the *Plasmodium* parasite, in particular in P. *berghei.* Such codon-optimized sequence encoding murine IL-6 is illustrated by the sequence of SEQ ID No.2. Codon-optimization (CO) may be performed in accordance with methods well known in the art including by using algorithms to derive the CO sequence from the native gene.

According to the invention, the expressed IL-6 protein (IL-6 cytokine) is biologically functional or active. Accordingly, the transgenic *Plasmodium* parasite expresses the IL-6 protein in a manner that elicits an immune-inflammatory reaction in the liver of the inoculated host and in addition elicits a specific adaptive immune response against the antigens of the *Plasmodium* parasite. In a particular embodiment, the expression of IL-6 induces or promotes the development of a long-lasting specific anti-parasite protection in the host. The immune response is said "specific" when it encompasses the elicitation of antibodies or immune active cells that are directed against or specifically respond to the antigenic repertoire of an infectious *Plasmodium* parasite.

A **"signal peptide"** and a **"nucleic acid encoding a signal peptide"** according to the invention relate to a peptide molecule, respectively a nucleic acid encoding such peptide molecule that enables the secretion of the encoded IL-6 from the transgenic *Plasmodium* parasite. Suitable nucleotide sequences encoding a signal peptide may be chosen from the group of the sequence encoding the signal peptide of P. *berghei* LISP1 protein, the sequence of SEQ ID No. 5 encoding the signal peptide of P. *berghei* LISP2 protein, the nucleic acid of sequence SEQ ID No.6 encoding the signal peptide MWWRLWWLLLLLLLLWPMVWA (SEQ ID No.7) and the nucleic acid of SEQ ID No. 8 encoding the signal peptide of human albumin (SEQ ID No. 9).

According to the invention, the composition of the transgene construct from 5' to 3' may involve in addition to the listed functional nucleic acid molecules encompassing the promoter, the sequence encoding the signal peptide and the sequence encoding the IL-6 protein, additional sequences necessary or advantageous for the recombination or for the cloning of said functional sequences, such as sequences of restriction sites for cloning or sequences for ligation. Apart from these additional sequences that do not convey the genetic information necessary for IL-6 expression, in a particular embodiment, the transgene construct may consist in the disclosed functional nucleic acid molecules in functional association.

**"EEF"** or "EEFs" "exoerythrocytic forms" of the *Plasmodium* parasite are in particular those arising from the parasite development that takes place in the liver of the infected host, in particular in hepatocytes. The elongate sporozoites (SPZ) (such as those inoculated by the mosquitoes during bloodfeed and provided as Mosquito-transmitted parasite stage to the host) are transported to the liver where they transform into spherical hepatic stages i.e. EEF, during a cellular remodeling process before achieving the stage of merozoites that will then reach the red blood cells for infection. Early and late EEFs are defined depending on their shape. Late EEF is defined herein as EEF that have achieved completely spherical forms such as obtained in hepatocytes of an infected mammalian host. Such forms may be characterized as expressing the HSP70 protein of the parasite in the bulb, preferably with cytoplasm compartimentalization of said protein or expressing the HEP17 protein.

**"genetically attenuated parasites"** or "GAP" as used herein designates genetically modified parasites which have lost at least in part their infectious potential for their host. GAP are known in the art and especially disclosed in Annoura, T. et al. (Assessing the adequacy of attenuation of genetically modified malaria parasite vaccine candidates. Vaccine 30, 2662-2670 (2012)). GAP may be obtained as sporozoites such as those delivered by SANARIA (USA).

A **"transgenic *Plasmodium* parasite"** of the invention is a genetically modified parasite obtained as a result of integration of a transgene construct of the invention into a *Plasmodium* parasite. The *Plasmodium* parasite may be originating from a wild-type strain or may be a genetically modified parasite, in particular GAP as defined herein. The transgenic *Plasmodium* parasite of the invention accordingly comprises a transgene encoding a biologically functional IL-6 cytokine, in particular a human IL-6 protein. It may be prepared using well known methods in the art (Nkrumah LJ et al, 2006), in particular by transfection with a plasmid vector bearing the transgene construct cloned therein, as illustrated in the Examples. The transfection may advantageously be a stable transfection. In a particular embodiment the *Plasmodium* parasite to provide the transgenic *Plasmodium* parasite of the invention is a genetically attenuated parasite resulting from the deletion of one or more parasite gene(s) providing an attenuation phenotype.

**"resistance against *Plasmodium* infection"** in a mammalian host according to the invention means that the infection of the host by a *Plasmodium* parasite is prevented or that the infection happens but is not followed by the development of the parasite toward all stages of its life cycle, in particular is not followed by the onset or the development of the erythrocytic stage of the parasite life cycle. The resistance to infection is accordingly achieved by impairing development of the *Plasmodium* parasite in the liver of the host, wherein such impairment is caused by increased level of local IL-6 expression by liver cells (T cells, macrophages, endothelial cells and hepatocytes). In particular, resistance against infection may also arise from the capability of the IL-6 expressed from the transgenic inoculated *Plasmodium* parasite to regulate the B and T lymphocytes activity to engage into parasite suppression. Accordingly, resistance against *Plasmodium* infection induced by the administration of the transgenic *Plasmodium* parasite of the invention may be related to the innate and/or adaptive immune response raised in the host following the expression of IL-6 from said transgenic *Plasmodium* parasite. The response raised may in particular encompass priming effector immune mechanisms to prevent the development of parasitemia. It may hence also result from impairment of the pre-erythrocytic replication of the parasite by an innate immunity-associated pro-inflammatory effector mechanism. In addition, the inventors have found that this innate-immunity-associated parasite clearance was followed by the establishment of a durable anti-parasite CD8⁺ T cell response. Accordingly, in a particular embodiment resistance against *Plasmodium* infection is the result of treatment of the mammalian host, in particular the human host, with the transgenic *Plasmodium* parasite provided to elicit IL-6 expression that brings or help bringing a specific humoral and/or cellular immune response against the *Plasmodium* parasite in the host.

In a particular embodiment, resistance against *Plasmodium* infection encompasses the preventive protection against the malaria disease in a mammalian, especially a human host. In a particular embodiment, resistance against *Plasmodium* infection encompasses conferring sterile protection of the mammalian, especially human host.

In a particular embodiment, resistance against *Plasmodium* infection encompasses liver stage immunity of the mammalian host, especially of the human host.

In a particular embodiment, resistance against *Plasmodium* infection encompasses the protection against acute forms of malaria disease, in particular against cerebral malaria, in a mammalian, especially a human host.

According to the invention, use for or method of raising resistance against *Plasmodium* infection in a mammalian host, in particular a human host, involves administering to such host an efficient protective, preferably prophylactic amount of the transgenic *Plasmodium* parasite according to the invention. Administration of the transgenic *Plasmodium* parasite according to the invention may be performed by exposure of the human host to mosquito bite when the mosquitos are infected with the transgenic *Plasmodium* parasite according to the invention or may be performed by administration of sporozoites of such transgenic *Plasmodium* parasite.

According to a particular embodiment, the transgenic *Plasmodium* parasite for use in the elicitation of a protective, preferentially prophylactic, immune response elicits antibodies directed against the antigenic polypeptides of the *Plasmodium* parasite, and/or elicits cellular response in the host need thereof, in particular a human host.

The invention also concerns the use of the transgenic *Plasmodium* parasite according to the invention in the preparation of a medicine for the preventive protection against *Plasmodium* infection in a mammalian host, in particular a human host, wherein the transgenic *Plasmodium* parasite is provided as a dose that, used in a single administration, or in an administration scheme encompassing multiple doses administration, elicits IL-6 expression to bring a specific immune response against *Plasmodium* parasite.

The invention also relates to a formulation or pharmaceutical composition, in particular a vaccine composition, suitable for administration to a mammalian host, in particular a human host, comprising a transgenic *Plasmodium* parasite according to the invention together with one or more pharmaceutically acceptable excipient(s) suitable for administration to a mammalian host in need thereof, in particular a human host.

Physiologically acceptable vehicles may be chosen with respect to the administration route of the immunization composition. In a preferred embodiment administration may be carried out by injection, in particular intramuscularly, intradermally, subcutaneously.

According to a particular embodiment of the use or the method of raising resistance against *Plasmodium* infection in a mammalian host, in particular a human host, if multiple doses of the immunogenic composition are administered to the host, these doses are separated in time by at least 1 week (including by 1 week), in particular at least 2 weeks (including by 2 weeks), in particular at least 6 weeks (including by 6 weeks), in particular at least 8 weeks (including by 8 weeks).

Doses of the administered transgenic *Plasmodium* parasite may range from 10⁵ to 10⁶ sporozoites, in particular 1.35x10⁵ to 9.0x10⁵ sprorozoites of said parasite administered as transgenic *Plasmodium* sporozoites.

The invention thus concerns a method of providing immunization in a mammalian host, especially in a human host, comprising the step of administering, as a prime or as a boost, the transgenic *Plasmodium* parasite to elicit the immune response, and optionally repeating the administration steps one or several times, in particular to boost said response, in accordance with the present disclosure. In a particular embodiment, the transgenic *Plasmodium* parasite is administered by administration of its sporozoites.

It has been observed by the inventors that in a particular embodiment, the transgenic *Plasmodium* parasite may be administered to the host without adding an adjuvant compound and/or without an immunostimulant compound. Indeed, the expression of the IL-6 protein by the transgenic *Plasmodium* parasite provides adjuvant or immunostimulant properties to the transgenic *Plasmodium* parasite.

### Legend of the Figures

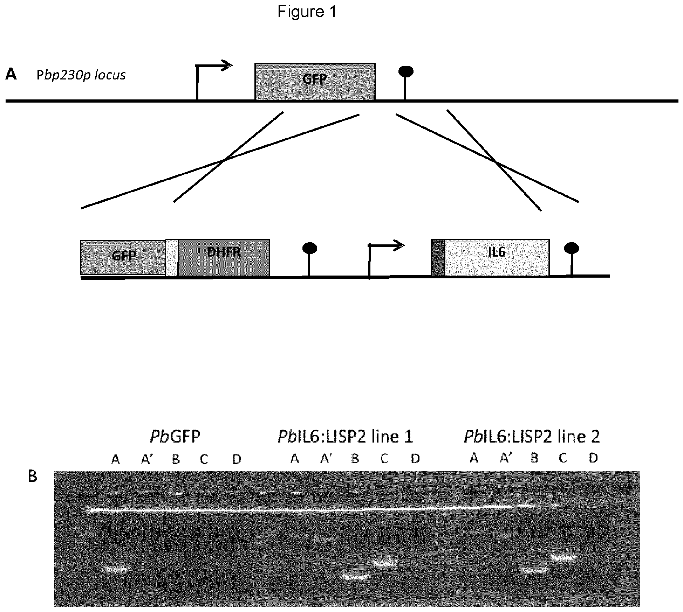

*Figure 1**:* Design of transgenic *Plasmodium berghei* parasites that express the mouse IL-6 cytokine
   A) Selection-linked integration strategy to replace the GFP cassette in *Pb*GFP parasites by a GFP-2A-hDHFR (SEQ ID No. 13) and mIL6 (SEQ ID No. 14) cassettes. B) PCR analysis of genomic DNA from parental *Pb*GFP, *Pb*IL6/LISP2 parasites (line 1 and line 2) . We used the following primer combinations: A (GFP/DHFRutrRev), which amplifies a 1020-bp band from WT locus and a 5007-bp band from recombined locus; B (GFPfor/DHFRseqRev), which amplifies a 292-bp band from WT locus and a 4641-bp band from recombined locus; C (GFPfor/huDHFRRev), which amplifies a 1397-bp only from recombined locus; D (IL6for/eEF1aPromTestRev), which amplifies a 1701-bp only from the plasmids. The results confirm the correct integration of the constructs at the GFP locus of *Pb*GFP parasites, and show the absence of parental parasites in the transfectants after a single round of pyrimethamine selection.

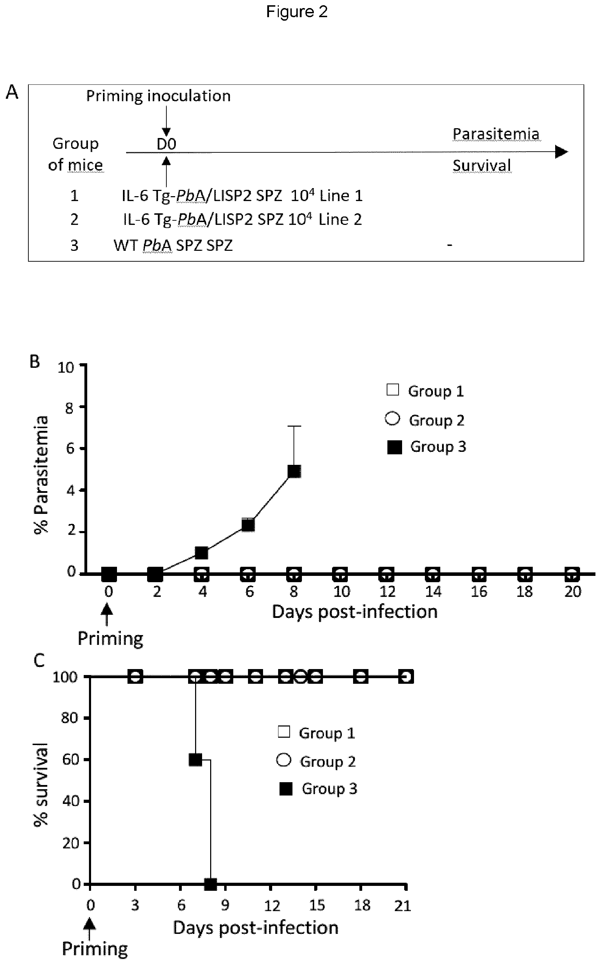

*Figure 2*: inoculation with IL-6 Tg-*Pb*A/LISP2 SPZ does not produce blood stage infections A) Three groups of C57BL/ mice were infected with 10⁴ IL-6 Tg-*Pb*A/LISP2 SPZ line 1 or with IL-6 Tg-*Pb*A/LISP2 SPZ line 2 or with WT PbANKA SPZ. B) Parasite development was measured at indicated time points by flow cytometry, as all parasites were GFP tagged. C) Survival rates were determined by Kaplan-Meier survival plots. Error bars, SEM. Data are representative of three independent experiments with 5 mice per group.

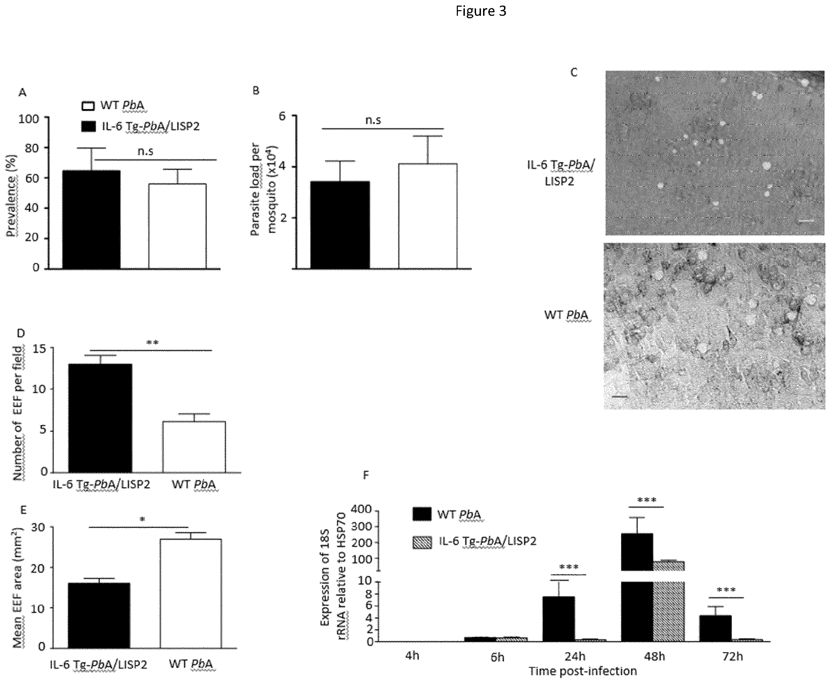

*Figure 3**:* IL-6 Tg-*Pb*ANKA/LISP2 did not show any major development defect in the vector life cycle and in cultured hepatocytes, in contrast to a deficient growth in the liver of infected mice.
   A, B) IL-6 Tg-*Pb*A/LISP2 parasites cycle normally between the anopheles and the vertebrate host. Cages of 200 IL-6 Tg-*Pb*A/LISP2- or WT *Pb*ANKA-infected *Anopheles stephensi* female mosquitoes showing GFP-labelled SPZ in their salivary glands were counted at day 20 post-blood feeding on infected C57BL/6 mice. Prevalence of infected mosquitoes was expressed as a ratio between positive ones and total mosquitoes (A). SPZ were extracted from salivary glands of 10 mosquitoes and counted. The number of SPZ was expressed as per pair of salivary glands (B). C) HepG2 hepatocytes were incubated together with IL-6 Tg-*Pb*ANKA/LISP2, or with WT *Pb*ANKA SPZ for 48h. Representative EEFs shown by fluorescence microscopy (Objective x 40), bars represent 100 µm. D) Quantitative representation of EEFs counted by fluorescence microscopy on 20 fields (objective x 10). Counts were performed on 4 wells per condition (Mann Whitney test; **p < 0.003). E) Quantitative representation of EEFs size. Mean EEF area of parasites was measured using Image J software (n=10-15 per group) (Mann Whitney test; *p = 0.03). F) Groups of C57BL/6 mice were injected i.v with 10⁴ WT *Pb*ANKA or with IL-6 Tg-*Pb*ANKA/LISP2 SPZ and livers were collected at indicated time points. Parasite loads in the liver were assessed by measuring parasite r18S mRNA by real-time RT-qPCR. Gene mRNA expression was normalized to the parasite control gene HSP70. Data are presented as the means ± SD from six individual values (***P < 0.001; Mann Whitney test).

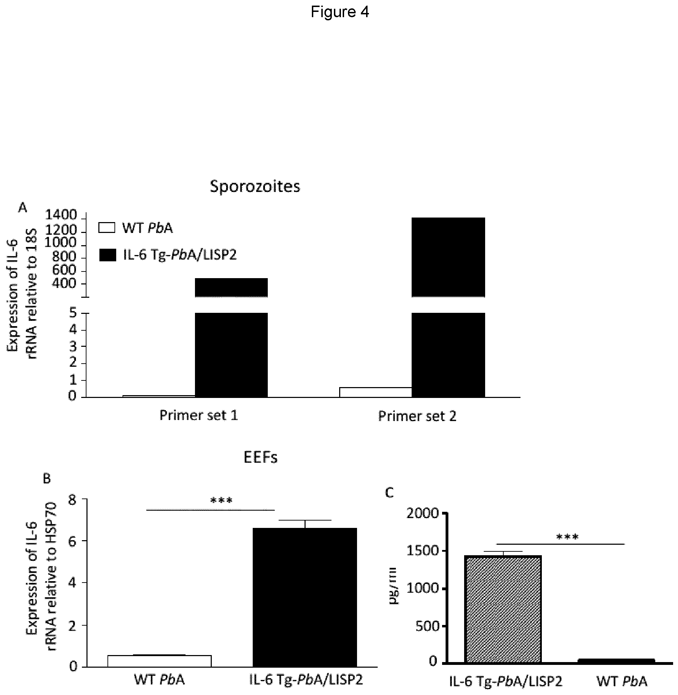

*Figure 4**:* IL-6 Tg-*Pb*ANKA/LISP2 parasites express IL-6 mRNA and produce the murine IL-6 cytokine
   A) WT PbANKA or IL-6 Tg-*Pb*ANKA/LISP2 SPZ collected from salivary glands of *Anopheles stephensi* were analyzed, using RT-qPCR, for IL-6 mRNA expression relative to 18S mRNA levels. B) After infection with WT *Pb*ANKA or IL-6 Tg-*Pb*ANKA/LISP2 SPZ, livers were collected at 48h post-infection, and RT-qPCR analysis was used to measure the IL-6 mRNA expressed by EEFs using a dedicated set of primers relative to the parasite control gene HSP70. C) Determination of IL-6 by ELISA in supernatants obtained from SPZ cultured in the presence of the HEpG2 hepatoma cell line for 48h shows that IL-6 was found to be secreted only in cultures with IL-6 Tg-*Pb*ANKA/LISP2 SPZ. Error bars, SEM. Data are representative of two independent experiments using triplicate samples (***p < 0.001; Mann Whitney test).

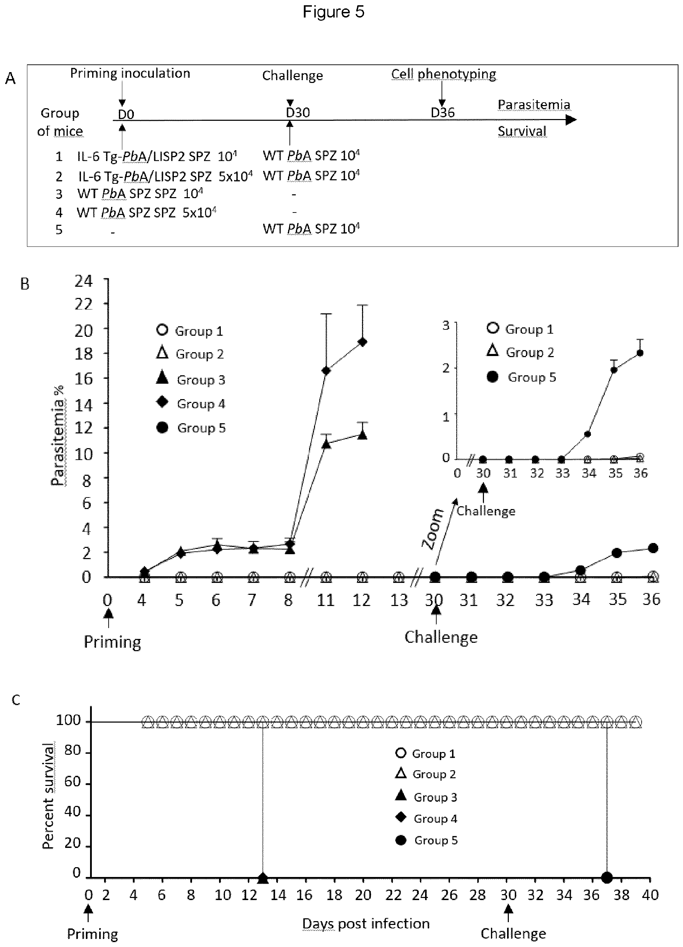

*Figure 5**:* a single inoculation with IL-6 Tg-*Pb*A/LISP2 SPZ is partially efficient in protecting mice against a lethal challenge
   A) Groups of C57BL/6 mice were infected with either 10⁴ or 5 x 10⁴ IL-6 Tg-*Pb*A/LISP2 SPZ or with the same doses of WT *Pb*ANKA SPZ. Mice were then challenged with 10⁴ *Pb*ANKA SPZ 30 days after priming. B) Parasite development was measured at indicated time points by flow cytometry, as all parasites were tagged with GFP. The insert is meant to highlight the magnification of the parasitemia to show a delayed and discrete breakthrough of parasitemia in IL-6 Tg-*Pb*A/LISP2 SPZ-treated groups. C) Survival rates were determined by Kaplan-Meier survival plots. Error bars, SEM. Data are representative of three independent experiments with 5 mice per group.

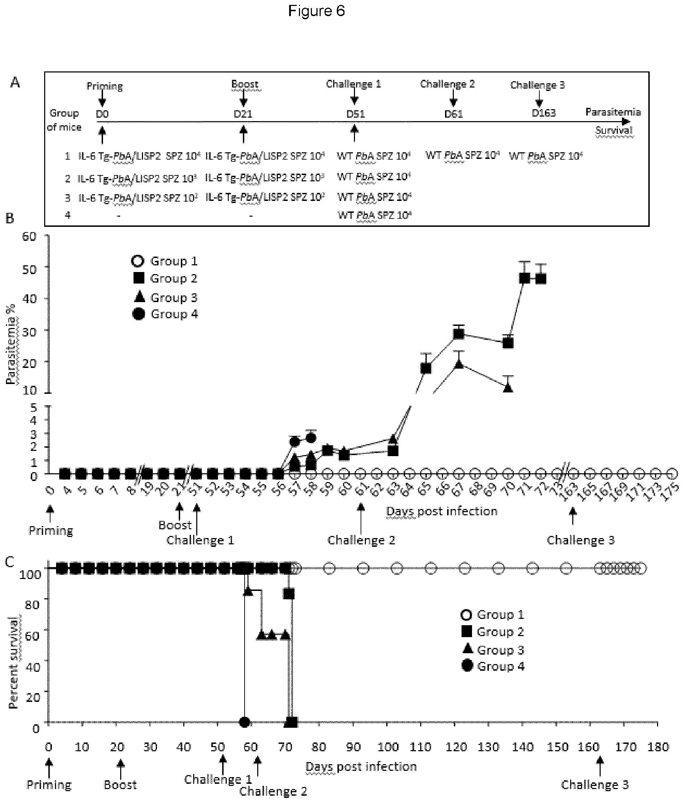

*Figure 6**:* homologous prime/boost immunization regimen with IL-6 Tg-*Pb*ANKA/LISP2 parasites confers a stable and efficient protection against challenge with WT *Pb*ANKA parasites
   A) Groups of C57BL/6 mice were infected with various doses of IL-6 Tg-*Pb*A/LISP2 SPZ and boosted with the same doses at 3 weeks interval. Mice were then challenged 30 days after the boost (day 51) with 10⁴ WT *Pb*ANKA SPZ. Mice of group 1, which all survived the lethal challenge, were then re-challenged at day 61 and day 163 with 10⁴ *Pb*ANKA SPZ. B) Parasite development was measured at indicated time points by flow cytometry, as all parasites were tagged with GFP. C) Survival rates were determined by Kaplan-Meier survival plots. Error bars, SEM. Data are representative of three independent experiments with 5 mice per group.

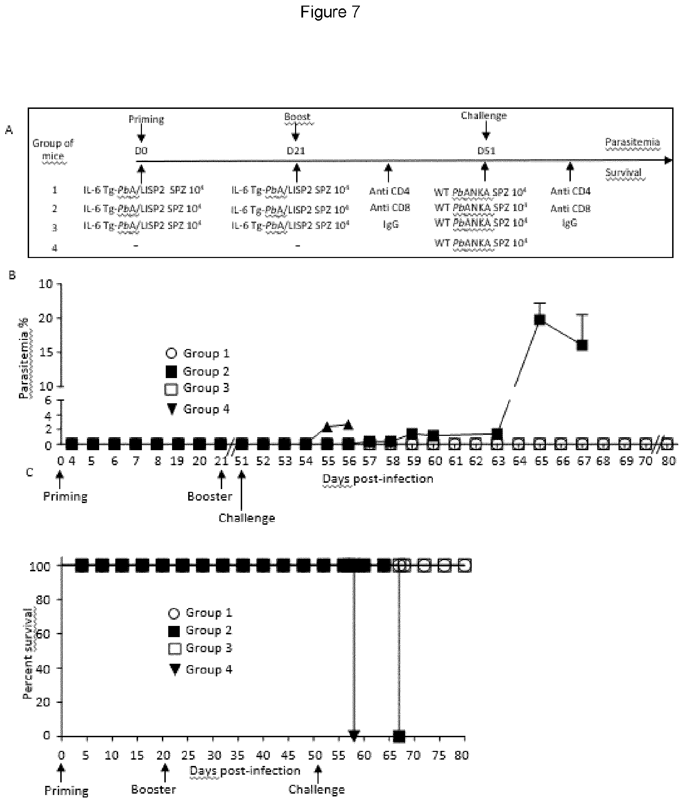

*Figure 7*: IL-6 Tg-*Pb*ANKA/LISP2 SPZ-mediated protection is dependent on CD8⁺ T cells
   A) Groups of C57BL/6 mice were infected twice with of 10⁴ IL-6 Tg-*Pb*A/LISP2 SPZ at 3 weeks interval, and then challenged 30 days later (day 51) with 10⁴ WT *Pb*ANKA SPZ. Two groups of mice were treated with either anti-CD4 (group 1) or anti-CD8 (group 2) depleting antibodies 2 days before and every other day after challenge. Control mice (group 3) were treated with non-specific IgG. B) Parasitemia was monitored over time by FACS analysis, as parasites were tagged with GFP. C) Survival rates were determined by Kaplan-Meier survival plots. Error bars, SEM. Data are representative of two independent experiments with 5 mice per group.

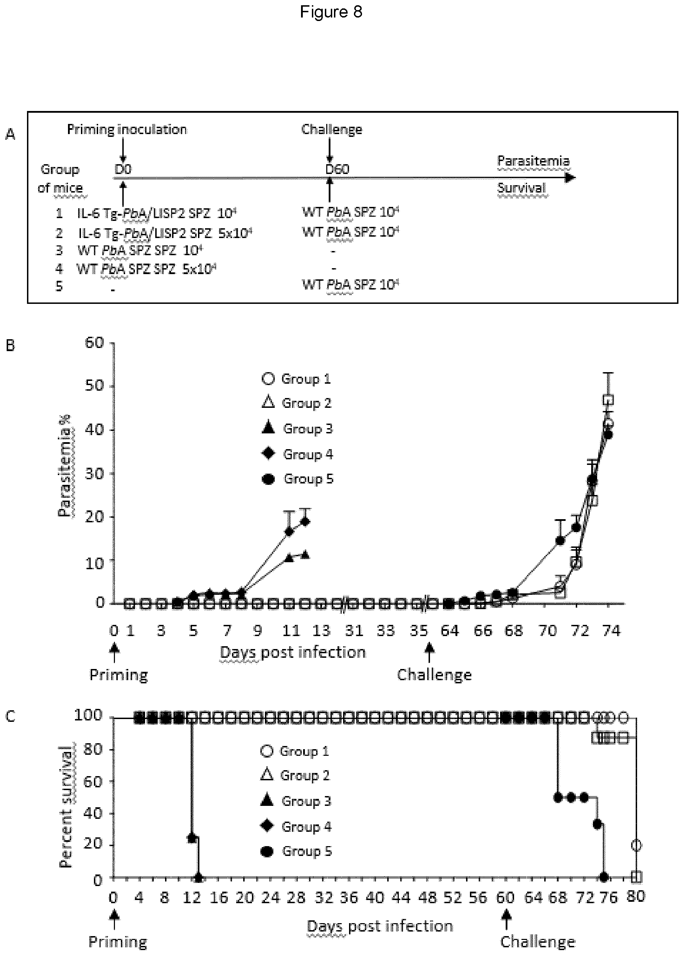

*Figure 8* : delayed challenge after priming with a single IL-6 Tg-*Pb*A/LISP2 SPZ inoculation is efficient but not fully protecting mice against lethal infection
   A) Groups of C57BL/6 mice were inoculated with either 10⁴ or 5 x 10⁴ IL-6 Tg-*Pb*A/LISP2 SPZ or with the same doses of WT *Pb*ANKA SPZ. Mice were then challenged with 10⁴ *Pb*ANKA SPZ 60 days after priming. B) Parasite development was measured at indicated time points by flow cytometry, as all parasites were tagged with GFP. C) Survival rates were determined by Kaplan-Meier survival plots. Error bars, SEM. Data are representative of three independent experiments with 5 mice per group.

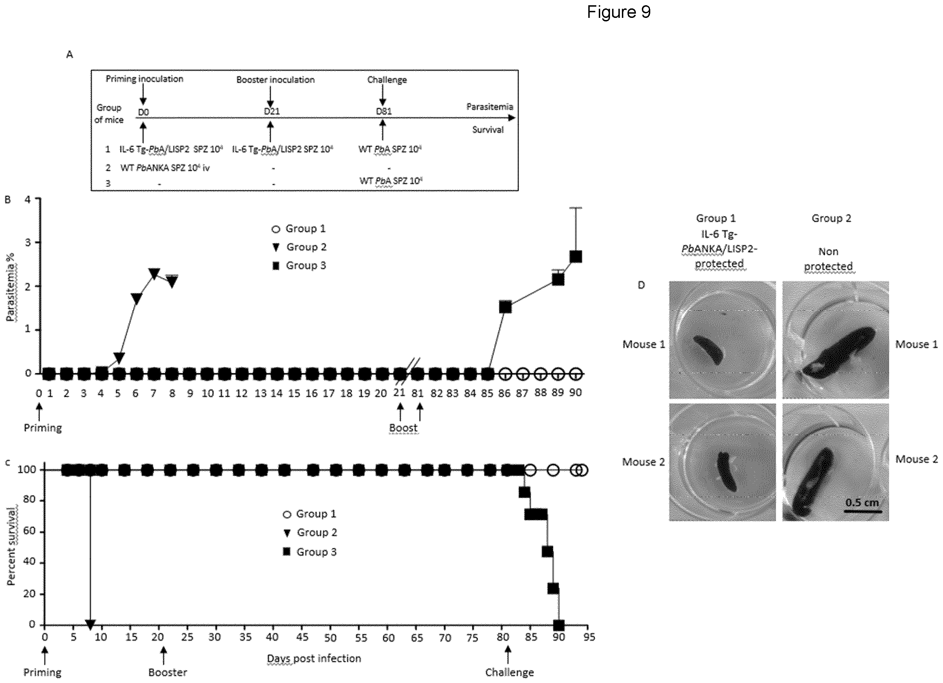

*Figure 9* : homologous prime/boost immunization regimen with IL-6 Tg-*Pb*ANKA/LISP2 parasites confers protection against temporally distant challenge with WT *Pb*ANKA parasites A) Groups of C57BL/6 mice were infected twice with 10⁴ IL-6 Tg-*Pb*A/LISP2 SPZ at 3 weeks interval or with the same dose of WT *Pb*ANKA SPZ as control. Mice were then challenged with 10⁴ *Pb*ANKA SPZ 60 days after the booster injection. B) Parasite development was measured at indicated time points by flow cytometry, as all parasites were tagged with GFP. C) Survival rates were determined by Kaplan-Meier survival plots. D) Macroscopic examination showed a more important splenomegaly at day 6 after challenge in WT-infected mice but not in IL-6 Tg-*Pb*A/LISP2 SPZ-protected mice. Error bars, SEM. Data are representative of three independent experiments with 5 mice per group.

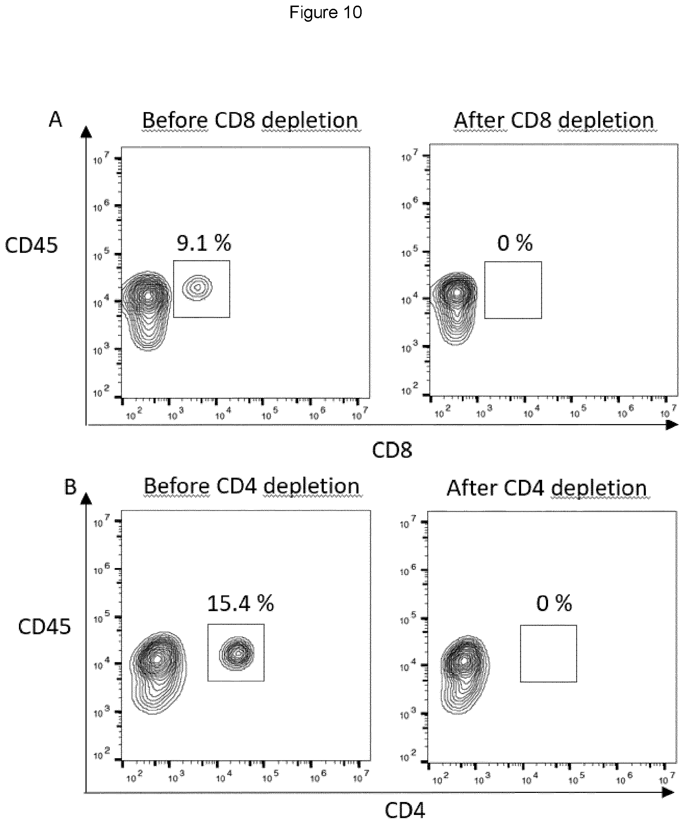

*Figure 10* : Assessment of leukocyte depletion.
   *In vivo* depletion of CD4⁺ or CD8⁺ T cells in protected mice using anti-CD4 or anti-CD8 depleting antibodies was assessed by measuring daily the percentage of residual (A) CD8⁺ or (B) CD4⁺ T cells in the blood by FACS analysis. Typical analysis performed at day 2 post treatment, corresponding to the day of challenge with WT *Pb*ANKA SPZ (refer to Fig. 7), is shown in this figure. Representative data from two independent experiments with 5 mice per group are shown.

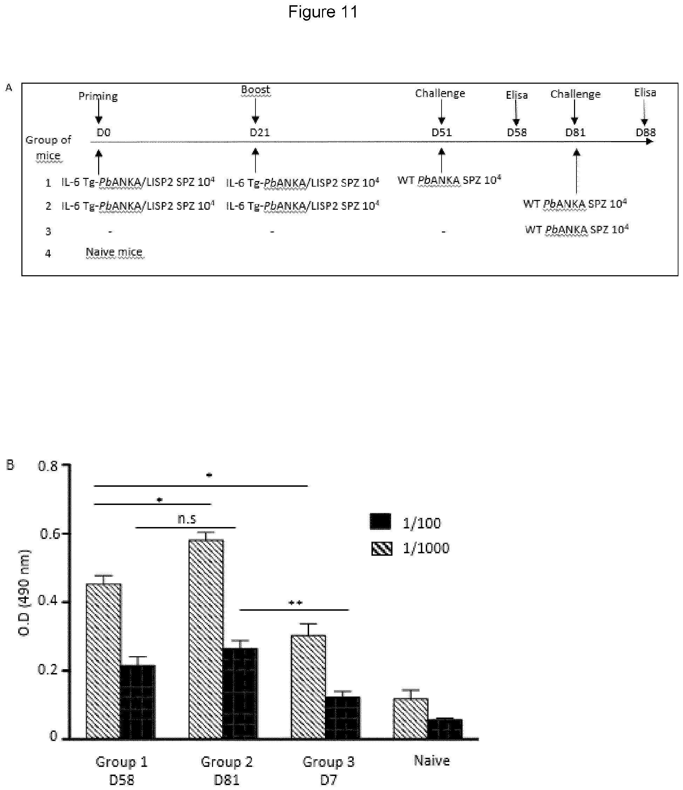

*Figure 11* : IL-6 Tg-*Pb*A/LISP2 SPZ-induced protection is associated with low but significant anti-SPZ IgG response
   (A) ELISA detection and quantification of serum anti-parasite-specific IgG antibodies was measured in mice subjected to various immunization protocols. B) Measurement by ELISA of anti-SPZ IgG antibodies in mouse sera at indicated time points. Error bars, SEM. Data are representative of two independent experiments with five mice per group. *, 0.02 < P < 0.03; **, P < 0.01; Mann-Whitney test.

**Table 1 : primer combinations to assess the integration of the murine IL-6 transgene into the PbANKA parasite genome.**

| Primer | SEQ ID No. | Sequence |
|---|---|---|
| **GFP fw** | 21 | CCGCCTAGGAAAATGAGTAAAGGAGAAGAACTTTTCACTGG |
| **DHFRutr Rev** | 22 | CGCAATTGTGAAATTAATAAAATAAAATACATATCCCTC |
| **DHFRseq Rev** | 23 | TGCATGCACAAAAAAAAATATGCACAC |
| **huDHFR Rev** | 24 | CCGGAATTCTTAAACACAGTAGTATCTGTCACCAAAG |
| **IL6 fw** | 25 | ACAAAATATGATTATTTTCCAACTTCACAAGTCAGAAGAGG |
| **eEF1PromTest Rev** | 26 | CCGCCTAGGTATAAAATTTTTATTTATTTATAAGC |

*Table 2:* Generation of transgenic *P. berghei* parasites expressing the murine IL6 transgene using a selection-linked integration strategy which enhances locus modification through binding to the selection marker. A) first cassette, B) second cassette.
A - **first cassette** (SEQ ID No. 13): 3' terminal sequence of the GFP coding sequence (bold upercase letters) (SEQ ID No. 16), fused to a 2A skip peptide (upercase letters in italics) (SEQ ID No. 17) and the human dihydrofolate reductase gene (lowercase letters) (SEQ ID No. 18), followed by the 3' UTR of P. berghei CAM (underlined upercase letters) (SEQ ID No. 19) 
CCATGGCCAACACTTGTCACTACTTTCGCGTATGGTCTTCAATGCTTTGCGAGATACCCAGATCATATGAAACAGCATGACTT
TTTCAAGAGTGCCATGCCCGAAGGTTATGTACAGGAAAGAACTATATTTTTCAAAGATGACGGGAACTACAAGACACGTGCTG
AAGTCAAGTTTGAAGGTGATACCCTTGTTAATAGAATCGAGTTAAAAGGTATTGATTTTAAAGAAGATGGAAACATTCTTGGA
CACAAATTGGAATACAACTATAACTCACACAATGTATACATCATGGCAGACAAACAAAAGAATGGAATCAAAGTTAACTTCAA
AATTAGACACAACATTGAAGATGGAAGCGTTCAACTAGCAGACCATTATCAACAAAATACTCCAATTGGCGATGGCCCTGTCC
TTTTACCAGACAACCATTACCTGTCCACACAATCTGCCCTTTCGAAAGATCCCAACGAAAAGAGAGACCACATGGTCCTTCTT
GAGTTTGTAACAGCTGCTGGGATTACACATGGCATGGATGAACTATACAAAGGTTCGGGAGAGGGCAGAGGATCCCTGCTAAC
 
ATGCGGTGATGTCGAGGAGAATCCTGGCCCAatggttggttcgctaaactgcatcgtcgctgtgtcccagaacatgggcatcg
gcaagaacggggacctgccctggccaccgctcaggaacgaatttagatatttccagagaatgaccacaacctcttcagtagaa
ggtaaacagaatctggtgattatgggtaagaagacctggttctccattcctgagaagaatcgacctttaaagggtagaattaa
tttagttctcagcagagaactcaaggaacctccacaaggagctcattttctttccagaagtctagatgatgccttaaaactta
ctgaacaaccagaattagcaaataaagtagacatggtctggatagttggtggcagttctgtttataaggaagccatgaatcac
ccaggccatcttaaactatttgtgacaaggatcatgcaagactttgaaagtgacacgttttttccagaaattgatttggagaa
atataaacttctgccagaatacccaggtgttctctctgatgtccaggaggagaaaggcattaagtacaaatttgaagtatatg
agaagaatgattaaATTATTAATATATATGAATATATATACATCGTTGTATGCGTAATATACGTGTAACTGTTTTTTTTAATT
TTTATATTAACTTTTCCTTATACATTTTAATGCCATTAACTAATAATATTTTACTTGAAAAAATTCTAAATTTTTATAACTCT
AATATAAAATCTGTGAATAATAAAAAATTAGCATAAAGTACGAATTAAACATCGAATATTTGTTTTAAAGGGGCACACATTAT
GTGCATATAATTTTATTCTTTGAATTTAATTTTTTGCTTATAATTAGATTAAATTAATATAATTTTTTTATTGAAAATAAAAA
CTTATTTTAAAAAATTAACAAATGGAAGTTAATGAAAAATACGAAAAAAGTAAAGGGTTAATTCTTATATGGTCATATAAAAA
AACAAATGAAATAATGTAAACAGTTATTCATTTTTTAAGGGTATAAAATAAGCTTATAAATAATGTTAGCATATTAAAAACAG
TGATATAAGTATGTGTAAATATGAAAGAATATAATAATGGATAGTTAAAAAAATATATTTTAAAGGGAATATTGACAAATTAT
ATACTGAAGCACTAAAGAAAATATCATCTTTGTATATTATAGCTTCACATTTCCAAGCAAATGGTGCCACAAATACATTCTTC
TCTCTCC
**B** - **second cassette** (SEQ ID No. 14) : sequence of signal peptide of *P. berghei* LISP2 (uppercase letters in italics) (SEQ ID No. 5), codon-optimized version of murine IL6 (bold uppercase letters) (SEQ ID No. 2), under control of the promoter of *P. berghei* LISP2 (bold lowercase letters) (SEQ ID No. 10) followed by the 3' UTR of *P. berghei* DHFR (underlined lowercase letters) (SEQ ID No. 20)

tggttaaatcgttttcacataaacgatgctatcaatttattaaagcatataataatataaaaaaaaagtatgatcaaaataaa
acaatattataaatgttcttaaacatcaaaaaatggaatatatacattaaaaatgtgcacatcttttaagtgttcattagcat
atatcatttttcgtatgatctttaaatctagaagtcctcctacacatgtgaatgtatatgtatataattttttaacgatgtaa
cagtgttgtatatattattgaagatatacattatttatgttaaaaaatcatgtaaacaaaattataggaataaactaattttg
aatcatttatttgcttaaaatttaaccacattatatttaataaatataaaataaaaatatgccacatccattctatactattt
attggtacatcgacattcgccataaaatatttcatagagttgcattatcgtcaaaagtgtataaaattataaaatagagcaac
ataaaatggataataatggtgaaactaaaaataataaaattaaagttgggtaaataaaaacacaacataaaggatcaaggtat
tatttttttaaattttttttatgaaaaactattagaacaatttaaaataatgcatattatgctttatttgtacaatataatta
aaataaaacgaataacgatgtgattattcaatatgcagattaataatatatacacatgtaaatgtatatatacttcttcattg
tcaaattccttaggcatatgtagcatatatggtttttgacggttctttaaaaatatacaactgtaatccctaataaatgaatg
cagttgaaatcgaacaaatccatatgcttccgtatcgatttaaccatttaggtagtgatctatgatcgatctacatgtgaaga
aaattggcatcttttaaaaactctttcataatggtttatgagttctttagatcatatattatttaaacaaaaaataaaataaa
tcacctatttttttatattcgataaagcgtgcaaacaaatttgcatgtatgtattctattatcgttggttttttttacaccac
cttttttttatttataatgtttttcaccacaaagcaactatatataaaaaacacactaaaaatatataattaaattaaaaaat
aataataaagaaaataaaaaataaaaaataaaaagcatatgtattatacatgcatgtatattcccctgcatatgtatgttttc
agttcagattaagaacaaaaaaatacagataattaagcatttatttgttacgtttatacatgtcaataccaaaaatatatatg
tgcctacacatgttacatataagtagatacatataaatatatcggagtaatattcttcttctttcatttttcacttctattat
aatcattttacttttttacacataaaaaATGAAAGATCATATAAAAAATGTATGCTTTCGGAAAACTCTACTAATTTCACTAC
TGCTTATTATATTAAAATACACAAAATATGATTATTTTCCAACTTCACAAGTCAGAAGAGGTGATTTTACAGAAGATACCACT
CCAAACAGACCAGTTTACACTACTTCTCAAGTTGGTGGTTTGATCACTCATGTTTTGTGGGAAATTGTTGAAATGAGAAAGGA
ATTGTGTAATGGAAATTCTGATTGTATGAACAACGATGATGCTTTGGCTGAAAATAATTTGAAGTTGCCAGAGATTCAAAGAA
ATGATGGATGTTATCAAACAGGATACAATCAAGAGATCTGTTTGCTGAAGATTTCTTCTGGATTGTTGGAGTATCATTCTTAT
TTAGAATACATGAAAAACAATTTAAAGGATAACAAGAAAGACAAAGCTAGAGTTTTACAGAGAGATACTGAAACTTTGATTCA
TATTTTCAACCAAGAAGTTAAAGATTTGCATAAAATTGTCCTTCCAACACCAATTTCAAATGCTTTATTGACTGATAAACTTG
AATCTCAGAAAGAATGGTTGAGAACTAAAACTATTCAGTTTATTTTAAAATCTTTGGAAGAATTTTTGAAAGTTACTTTAAGA
TCTACTAGACAAACTTAACTCGAGGAATTCtcgtttttcttatttatatatttataccaattgattgtatttataactgtaaa
aatgtgtatgttgtgtgcatatttttttttgtgcatgcacatgcatgtaaatagctaaaattatgaacattttattttttgtt
cagaaaaaaaaaactttacacacataaaatggctagtatgaatagccatattttatataaattaaatcctatgaatttatgac
catattaaaaatttagatatttatggaacataatatgtttgaaacaataagacaaaattattattattattattatttttact
gttataattatgttgtctcttcaatgattcataaatagttggacttgatttttaaaatgtttataatatgattagcatagtta
aataaaaaaagttgaaaaattaaaaaaaaacatataaacacaaatgatgttttttccttcaatttcgatatcatatagcaatc
gtattagcaggtaaatgagcaaggttaataaaaaaatggtaaaaaaaaaaaaattggggaaaaataaacaaggtgttctttat
ctacactttggctttacaatgtataatttatatgcatacaaaaaaattttgataaaattgtaaaaaggtgaataaaataacat
tgtaacagtagtagatagtaaagggaaggtgttgctcaaatagtgtcgaaacaaaactggcataaagaaaaattgatattgag
cagaggatatgcgcataatggtatttgtttgtttgttttctcattttttgagacagctcaattctttatgtccacaac
atcatcggacttttcttcttca

**Table 3: list of oligonucleotides used for RT-qPCR analyses to assess parasite load and to quantify IL-6 expression**

| **Primer** | **SEQ ID No.** | **Fw/Rev** | **Sequence** |
|---|---|---|---|
| **Pb18S** | 27 | **Fw** | ATTAATCTTGAACGAGGAATGGCT |
| | 28 | **Rev** | TCAATCGGTAGGAGCGACG |
| **Pb LISP2** | 29 | **Fw** | GCCAAATGCTAAACCTAATG |
| | 30 | **Rev** | TGGGTTTGTATTGTATGCAC |
| **Pb HSP70** | 31 | **Fw** | TGCAGCTAATCAAACTC |
| | 32 | **Rev** | ACTTCAATTTGTGGAACACC |
| **Mu HPRT** | 33 | **Fw** | CTGGTGAAAAGGACCTCTCG |
| | 34 | **Rev** | TGAAGTACTCATTATAGTCAAGGGCA |
| **Mu IL6** | 35 | **Fw** | AAAGAAATGATGGATGCTACCAAAC |
| | 36 | **Rev** | CTTGTTATCTTTTAAGTTGTTCTTCATGTACTC |

Further aspects of the invention and features of the disclosed embodiments will be disclosed in the following Examples and corresponding figures.

### EXAMPLES

Protozoan pathogens use a number of different strategies to avoid host immune destruction. In malaria parasites (*Plasmodium* parasites), antigenic variation has been shown to contribute to chronic infection. However, this mechanism alone cannot explain the prolonged chronic infections observed in human malaria patients. Recent findings using murine models demonstrate that *Plasmodium berghei* (*Pb*) malaria parasites as well as the human *Plasmodium falciparum* parasites selected genes that are endowed with immuno-suppressive (tolerance) properties.

One of these genes called HRF (Histamine Releasing Factor) was found to be endowed with immuno-suppressive properties: in particular the HRF protein down-regulates IL-6 production in the liver of the host and thereby facilitates normal parasite development. In contrast, infection with HRF-deficient parasites elicits a sharp and rapid rise in the IL-6 production in the liver of the host which resulted in an abortive parasite development and a subsequent establishment of a long-lasting anti-parasite immunity.

Using an alternative model, the inventors proposed that the nature and the magnitude of the inflammatory response within the host liver tissue is key for the establishment of the infection. Liver inflammation can be induced in many ways, including by host genetic disorders or drug taking. The inventors used mice deficient in the multidrug resistance-2 gene (Mdr2^{-/-}) which encodes the canalicular phospholipid flippase leading to a complete absence of phospholipids from bile, and which spontaneously develop liver inflammation ultimately leading to a low-grade hepatitis. Using this model, the inventors have shown that in contrast to wild type mice, *Plasmodium berghei-infected* Mdr2^{-/-} mice did not develop blood stage parasites following challenge with sporozoites (SPZ). Accordingly, intravital microscopy and qPCR data showed a significant decrease of exo-erythrocytic forms of the parasite in Mdr2^{-/-}mice at early time points after challenge, suggesting that parasites are blocked inside the liver. The abortive infection led to the priming of the immune system of Mdr2^{-/-} mice and resulted in a long-lasting immune protection against both SPZ and blood-stage infections where neutrophils and IL-6 were found to be key effector components. Interestingly, *ex vivo* infections of primary hepatocytes from Mdr2^{-/-} mice indicate that these hepatocytes are permissive to parasite development, suggesting that the pre-erythrocytic stage development is hampered by an innate immunity-associated pro-inflammatory effector mechanism. The inventors have now found that this innate-immunity-associated parasite clearance was followed by the establishment of a durable anti-parasite CD8⁺ T cell response. The data provided herein demonstrate the critical role of inflammation in the breakdown of liver tolerance and the generation of anti-parasite immunity and provide new approaches for the treatment of malaria infections. Here again, early production of IL-6 response was found to be essential in the resistance to the parasite infection.

Approaching the parasite infection in mice from different angles, and using either mutant parasites (*Pb*HRFΔ) or genetically altered hosts (MDR2^{-/-}), it is rather surprising that common signaling pathways, namely IL-6 production, was constantly found as a key mechanism that not only block parasite development, but more importantly allows the initiation of a robust and long-lasting anti-parasite immunity. This is probably an evolutionary conserved mechanism that evolved to eradicate the infection and against which the parasite has selected genes, such as HRF, making it more elusive with regard to the host immune system.

### Examples and experimental data:

### Preparation of a Plasmodium parasite transgenic for IL-6 expression and use in assessing protection against Plasmodium infection in a suitable animal model

In order to assess the efficiency of the designed construct *Plasmodium berghei* strains transgenic for the murine IL-6 gene are designed. The advantage of this approach is that the *Pb*-IL-6 transfectant is self-sufficient in that it does not need to be delivered in association with a distinct adjuvant, since it both provides the antigenic repertoire and encodes for the IL-6 that can be considered here as the most potent adjuvant of the immune response.

Considering the specificities of host sensibilities for infection by *Plasmodium* parasite strains the inventors adopted two options:
1- **Transfection of the wild-type *Pb*ANKA strain** with the mouse IL-6 gene: this design enabled study of the effect of administration of the transgenic *Plasmodium* strain in an animal model (mouse) sensible to infection by *P. berghei .*
2- **Transfection of the wild-type** *Plasmodium falciparum* parasites infecting human with the human IL-6 gene.

### I. Materials and methods

### Ethics statement

All animal care and experiments described in the present study involving mice were conducted at the Institut Pasteur, approved by the "comité d'éthique en expérimentation animale" (CETEA) (Permit Number N° dap180040 issued on 2018) and performed in compliance with institutional guidelines and European regulations. A statement of compliance with the French Government's ethical and animal experiment regulations was issued by the French "Ministère de l'Enseignement Supérieur et de la Recherche" under the number 00218.01.

### Design of transgenic Plasmodium berghei parasites that express the mouse IL-6 cytokine

To generate transgenic *P. berghei* parasites expressing the murine IL6 transgene, we used a selection-linked integration strategy (Birnbaum et al., 2017). We first assembled two plasmids, named GFP-SLI-LISP2 and GFP-SLI-IL6, both containing two cassettes. The first cassette includes a 3' terminal sequence of the GFP coding sequence, fused to a 2A skip peptide and the human dihydrofolate reductase gene, followed by the 3' UTR of *P. berghei* CAM (Figure 1A, **Table 2A**). The second cassette corresponds to a codon-optimized version of murine IL6, under control of the promoter of *P. berghei* LISP2 (in the GFP-SLI-LISP2 plasmid), and followed by the 3' UTR *of P. berghei* DHFR. To ensure IL6 secretion, the peptide signal peptide of mIL6 coding was replaced by the signal peptide *of P. berghei* LISP2 in the GFP-SLI-LISP2 plasmid (**Table 2B**). The final construct was verified by DNA sequencing before linearization for transfection. Linearized plasmid constructs were used to transfect GFP-expressing *P. berghei* (*Pb*GFP, ANKA strain) parasites (Manzoni et al., 2014). *Pb*GFP-infected erythrocytes were collected from an infected donor mice and cultured overnight to allow parasite maturation into erythrocytic merozoites. Merozoites were transfected by nucleofection, as described (Janse et al., 2006), and immediately injected into mice. One day after transfection, mice were administered pyrimethamine in the drinking water to select for recombinant parasites. Once the parasitemia reached 2%, after one week, infected erythrocytes were collected and DNA extracted for genotyping by PCR (Figure 1B) using primer combinations shown in **Table 1.** PCR analysis of genomic DNA from *Pb*IL6/LISP2 parasites confirmed correct integration of the constructs and the absence of residual parental parasites. Thus, after a single round of pyrimethamine selection following transfection, we could obtain pure populations of transgenic *Pb*IL6/LISP2 parasites.

### Parasites and mice infection

Seven- to eight-week-old female C57BL/6J Rj mice were purchased from Janvier Laboratories (Le Genest-Saint-Isle, France), and were maintained at the animal facility of Institut Pasteur. Mice were infected with either WT *Pb*ANKA SPZ or with IL-6 Tg-*Pb*ANKA/LISP2 SPZ collected from salivary glands of infected *Anopheles stephensi.* Infections were performed via i.v. injection in the tail vein of an indicated amount of SPZ according to the experiments or by natural infection by infectious mosquito bites. The natural infection was performed by the exposure of mice to bites of 10 infected *Anopheles stephensi* for fifteen minutes. Mosquitoes were provided by the CEPIA (Centre d'élevage, de production et d'infection des anopheles, Institut Pasteur, Paris - France). Survival and parasitemia as determined by FACS using Cytoflex cytometer (Beckman Coulter Life Sciences, Villepinte, France) and the software FlowJo (FlowJo LLC, Ashland, OR, USA) were then monitored daily, beginning day 4 p.i. Symptoms associated with the experimental cerebral malaria (ECM) in mouse models include coat ruffling, a respiratory distress syndrome, a drop in body temperature, and neurological signs such as paralysis, and coma, followed by death. For ethical reasons, manifestation of signs such as coat ruffling and reduced motor skills which represent a limit point, constitutes a criterion for interrupting the experience.

### Sporozoite development in HepG2 cells

HepG2 cell line, routinely tested negative for mycoplasma (PlasmoTest, InvivoGen, San Diego, CA), were thawed in complete DMEM medium containing 10% FCS and 2% Streptomycin/Neomycin and plated at a concentration of 4 x 10⁴ cells per well. WT *Pb*ANKA or IL-6 Tg-*Pb*ANKA/LISP2 SPZ purified from salivary glands were used for HepG2 infection at a ratio of 1:1 (parasite/cells) for 48 h, at 37°C, 5% CO₂ in the presence of PSN (penicillin-streptomycin-neomycin solution, Sigma). Parasite liver stage development was followed and quantified by counting the whole well by fluorescent microscopy using a Zeiss Axiovert microscope equipped with phase-contrast and epifluorescence.

### Preparation of total RNA and RT-qPCR analysis of mRNA

For kinetic analysis of *in vivo* parasite load, livers of C57BL/6J mice infected with WT *Pb*ANKA or with IL-6 Tg-*Pb*ANKA/LISP2 SPZ were surgically removed 4h, 6h, 24h, 48h, and 72h p.i., respectively. Total RNAs were extracted from the liver samples using the guanidinium-thiocyanate-phenol-chloroform method (all from Invitrogen, Waltham, MA, USA). RNA was thereafter reverse transcribed by PCR (temperature profile: 65°C for 5 min, 42°C for 50 min, and 70°C for 15 min) using 100 U of SuperScript II reverse transcriptase (Invitrogen, Waltham, MA, USA), 40 U RNase inhibitor, and 2 µM oligo(dT) 18S, HSP70, or LISP-2 rRNA primers (Eurofins MWG Operon) per sample. For detection of transcribed IL-6 mRNA by SPZ or EEF, the same procedures were applied. Expression levels of diverse transcripts were analyzed by real-time RT-qPCR using Power SYBR green PCR master mix (Applied Biosystems) and various primer sets (**Table 3**). All reactions were performed in a real-time PCR machine (temperature profile: 50°C for 2 min, 95°C for 10 min, 40 cycles of 15 s at 95°C, and 60°C for 1 min; ABI PRISM 7000 Sequence Detection System; Applied Biosystems). The relative abundance of parasite rRNA, or IL-6 mRNA in the liver and in SPZ was calculated using the ΔCt method and expressed as 2-*Δ*Ct. The mouse hypoxanthine phosphoribosyltransferase (HPRT) gene was used as an internal control for the variation in input RNA amounts. A no template control was included to ensure that there was no cross-contamination during sample preparation.

### Detection of specific antibodies, and the IL-6 cytokine

To detect parasite-specific antibodies, 96-well plates (Nunc-immuno plate; Thermo Scientific, Rockford, IL) were coated with parasite protein extract from SPZ in carbonate buffer, pH 9.6, for 2 h at 37 °C. After the plates were saturated with 1% (w/v) pork gelatine, each serum was assayed at serial dilutions and incubated for 2 h at 37 °C. Specific binding was detected using HRP-conjugated goat anti-mouse secondary antibody (Cell Signalling technology^{®}, Danvers, MA) followed by the addition of o-phenylenediamine dihydrochloride (OPD) substrate (Sigma-Aldrich; St.Louis, MO). Hydrogen chloride (HCl) 1 N was used to block the reaction. The optical density (OD) was read at 490-655 nm. Each sample was tested against non-immune serum and PBS as background controls. Amounts of IL-6 in supernatants of SPZ cultured alone or in the presence of HepG2 hepatocytes were analyzed by cytokine-specific ELISA kits (BD Biosciences, Mountain View, CA).

### In vivo cell depletion

To determine if the protection induced by IL-6 Tg-*Pb*ANKA/LISP2 SPZ is dependent on effector CD4⁺ or CD8⁺ T cells, cell-specific depletion experiments were performed. C57BL/6 protected mice were injected i.p. with 20 µg of anti-CD8 clone 53-6.7 Armenian hamster IgG (eBioscience, San Diego, CA) or 100 µg of rat anti mouse CD4 clone GK1.5 (ATCC^{®} TIB207^{™}) 48 h before the infection with *Pb*NK65 WT followed by 6 injections administered every other day after the infection. The cell depletion was followed and confirmed every day by taking 10 µl of blood from the tip of the mouse tail and analysed by flow cytometry. Blood samples were labeled with anti-CD45-AF 647 (clone 30-F11), anti-CD8a-PE (clone 5H10) from Biolegend (San Diego, CA), and anti-CD4-FITC (clone H129-19 from BD Bioscience, Mountain View, CA).

### Statistical analysis

All data were analyzed using Prism 5.0 software (GraphPad Software, San Diego, USA). Unpaired data between two groups at a specific time point were analyzed by a Mann-Whitney test for nonparametric analysis. Kaplan-Meier survival plots were analyzed using a Mantel-Cox test. A p-value <0.05 was considered to be statistically significant. All experiments were replicated several times as indicated in the figure legends.

### II. IL-6 transfected PbANKA parasite strains under the control of LISP2 promoter (IL-6 Tg-PbANKA/LISP2) fail to develop beyond the liver stage

To examine the capacity of IL-6 transgenic parasites to develop inside hepatocytes and to differentiate into blood stages, we used a selection-linked integration strategy (Birnbaum et al., 2017) where the murine IL-6 gene was expressed under the control of the *Plasmodium* liver-specific protein 2 (LISP2) (Fig. 1A). Two transfectants were selected from independent transfection experiments, called IL-6 Tg-*Pb*ANKA/LISP2 line 1 and line 2 and were verified, by using the primer combinations shown in Table 1, to harbour the expected integration of the murine IL-6 gene in a similar way (Figure 1B). To analyse the profile of infection of the IL-6 transgenic parasites, C57BL/6 mice were infected i.v with the dose of 10⁴ SPZ of IL-6 Tg-*Pb*ANKA/LISP2 line 1, or line 2, or with WT *Pb*ANKA SPZ, as shown in Fig. 2A, and parasitemia was followed over time. Parasitemia was determined by FACS by counting fluorescent red blood cells because of the presence of the GFP gene integrated into the parasite genome. As shown in Fig. 2B, we could observe that while mice infected with WT *Pb*ANKA parasites developed parasitemia, those mice which were infected with IL-6 Tg-*Pb*ANKA/LISP2 line 1 and line 2 parasites all failed to show any detectable parasitemia over a period of 20 days of follow up. Survival curves paralleled those of parasitemia in that mice infected with WT *Pb*ANKA parasites developed experimental cerebral malaria and died around day 8 post-infection (Fig. 2C) whereas all mice infected with either IL-6 Tg-*Pb*ANKA/LISP2 line 1 or line 2 survived. These data demonstrate that insertion of the mouse IL-6 gene in *Pb*ANKA parasites under the promoter of the late liver stage LISP2 gene resulted in the blockade of the parasite development. From now on, only the IL-6 Tg-*Pb*ANKA/LISP2 line 1 will be used throughout the study.

### III. IL-6 Tg-PbANKA/LISP2 did not show any major development defect in the vector life cycle and in cultured hepatocytes, in contrast to a deficient growth in the liver of infected mice

To assess whether IL-6 transgenic parasites have any developmental defect within the vector, when transmitted to female *Anopheles* mosquitoes, prevalence of mosquito infection (Fig 3A) and SPZ numbers (Fig 3B) of IL-6 Tg-*Pb*ANKA/LISP2 and WT PbANKA SPZ per infected mosquito salivary gland were found to be similar, indicating that transfected mouse IL-6 gene does not alter parasite development inside mosquitoes.

To investigate whether the impairment of parasite development in the liver of infected mice represents an intrinsic defect of the parasite growth or whether the production and the secretion of the IL-6 cytokine by the parasite machinery is responsible for the failure of the parasite development as a consequence of the involvement of the immune system, HepG2 cell lines were co-cultured either with IL-6 Tg-*Pb*ANKA/LISP2, or with WT *Pb*ANKA SPZ. As shown in Fig. 3C, all parasites were able to invade and develop inside HepG2 hepatocytes, however, counts of EEFs revealed that at 48h of culture, EEF counts of IL-6 Tg-*Pb*ANKA/LISP2 parasites were significantly higher than that of WT *Pb* ANKA parasites (Fig. 3D). Looking at the size of EEFs, the calculated mean EEF area at 48h of culture of IL-6 Tg-*Pb*ANKA/LISP2 was significantly lower than that of WT *Pb*ANKA parasites (Fig. 3E). To analyse liver-stage development *in vivo,* mice were injected with SPZ i.v and liver samples collected at indicated time points were subjected to RT-qPCR analysis of parasite 18S rRNA (Fig. 3F). At early time points 4h, 6h and 24h, no measurable IL-6 Tg-*Pb*ANKA/LISP2 parasites were observed, whereas WT *Pb*ANKA parasites were detected at 24h post-infection. It is only at 48 h after infection that the amount of IL-6 Tg-*Pb*ANKA/LISP2 parasites had risen but to levels by far lower than that of WT *Pb*ANKA parasites, 76.9 versus 255-fold increase. At 72h post infection, WT *Pb*ANKA parasites declined at levels close to the limit of detection whereas IL-6 Tg-*Pb*ANKA/LISP2 parasites were absent. Therefore, RT-qPCR analysis in the liver indicated that the development of the IL-6 Tg-*Pb*ANKA/LISP2 parasites was not only delayed but also occurs at a much lower level than that of the of WT PbANKA parasites.

These results demonstrate that transfection with the murine IL-6 gene does not affect the ability of cultured HepG2 hepatocytes to support EEF development and suggest that the failure of IL-6 Tg-*Pb*ANKA/LISP2 parasites to develop in the liver of infected mice is more likely due to an active IL-6-mediated anti-parasite immune response rather than to the inability of mutant parasites to invade and infect hepatocytes.

### IV. IL-6 Tg-PbANKA/LISP2 parasites express IL-6 mRNA and secrete the murine IL-6 cytokine

To search for the expression of the IL-6 gene at the transcriptional level, we quantified the IL-6 mRNA by RT-qPCR by using two sets of primers designed to detect codon-optimized murine IL-6 gene expressed by SPZ. As shown in Fig. 4A, IL-6 mRNA could be measured only in IL-6 Tg-*Pb*ANKA/LISP2 SPZ but not in control WT *Pb*ANKA SPZ. To assess whether developing EEFs express the IL-6 gene in infected mice, the liver of C57BL/6 mice inoculated with 10,000 SPZ of either IL-6 Tg-*Pb*ANKA/LISP2 or with WT PbANKA parasites were harvested at 48h post-infection and were subjected to RT-qPCR. As shown in Fig. 4B, IL-6 mRNA could be detected only in mice infected with IL-6 Tg-*Pb*ANKA/LISP2 parasites but not in those infected with WT *Pb*ANKA parasites. To verify whether the IL-6 Tg-*Pb*ANKA/LISP2 parasites secrete the IL-6 cytokine, supernatants were collected 48h after the HepG2 hepatocyte cell line was cultured in the presence of IL-6 Tg-*Pb*ANKA/LISP2, or with WT *Pb*ANKA SPZ. IL-6 measured by ELISA was present only in supernatants from IL-6 Tg-*Pb*ANKA/LISP2 / HepG2 cultures, but not from WT *Pb*ANKA / HepG2 cultures (Fig. 4C). These data demonstrate that IL-6 transfectants are able not only to express IL-6 mRNA *in vitro* and *in vivo,* but also to secrete IL-6 and that the IL-6 transgene is fully functional.

### V. Priming followed by a booster immunization regimen with IL-6 Tg-PbANKA/LISP2 parasites confers protection against challenge with WT PbANKA parasites

We decided to investigate whether the abortive development of SPZ into blood stage parasites in mice infected with IL-6 Tg-*Pb*ANKA/LISP2 parasites will result in adaptive immune protection against a challenge with WT *Pb*ANKA SPZ. A protocol of immunization shown in Figure 5A represents different groups of mice where we explored a dose effect at the priming stage with IL-6 Tg-*Pb*ANKA/LISP2 SPZ using either 10⁴ SPZ (groups 1) or 5 x 10⁵ SPZ (groups 2) followed by a challenge with 10⁴ WT *Pb*ANKA SPZ at day 30 post priming. Different control groups of mice were used including mice infected with 10⁴ or 5 x 10⁵ WT *Pb*ANKA SPZ at day 0 (groups 3 and 4) and mice which received 10⁴ WT *Pb*ANKA SPZ at day 30 (group 5). Before challenge, only mice that received WT *Pb*ANKA SPZ developed parasitemia and died (Groups 3 and 4, Fig. 5 B, C). For the challenge with WT *Pb*ANKA SPZ at day 30 post priming, parasitemia determined over time (Fig. 5B) indicates that only mice that received WT *Pb*ANKA SPZ (group 5) developed parasitemia and did not survive (Fig. 5C) whereas mice which received whatever dose of IL-6 Tg-*Pb*ANKA/LISP2 (Groups 1 and 2) did show only a very low parasitemia at day 6 post-challenge with a delay in the pre-patent period of 2 days (day 36, highlighted in the insert) as compared to control mice infected with WT *Pb*ANKA SPZ (Group 5). This delay represents approximately 2 Log difference (100 fold) in parasite load between control and protected mice, and consistent with this, no mice which received IL-6 Tg-*Pb*ANKA/LISP2 SPZ died from ECM (Fig 5C). These data demonstrate that one single priming with IL-6 transgenic *Pb*ANKA/LISP2 parasites was efficient in protecting mice against disease expression but not sufficient to induce sterile protection. Similar observation was made using the same protocol of priming except that the challenge with WT *Pb*ANKA SPZ occurred at 60 days post priming (Fig 8A). The clinically IL-6 Tg-*Pb*ANKA/LISP2 SPZ-protected mice (Groups 1 and 2) died however from hyperparasitemia (Fig 8B, C).

It looks as if one single inoculation with IL-6 Tg-*Pb*ANKA/LISP2 SPZ, even at the high dose of 5 x 10⁴ SPZ, was not sufficient to trigger an efficient priming of effector immune mechanisms to generate a sterile immunity following SPZ challenge with WT parasites. To optimize the immunization protocol, further experiments were developed in which mice were inoculated twice with 10⁴ IL-6 Tg-*Pb*ANKA/LISP2 SPZ at three weeks interval, and then challenged 30 days (Fig. 6A) or 60 days (Fig 9A) later with the same dose of 10⁴ WT *Pb*ANKA SPZ. As shown in Figure 6B, mice which received IL-6 Tg-*Pb*ANKA/LISP2 SPZ at the dose of 10⁴ parasites (group 1) not only did not show any parasitemia after the priming phase but more interestingly did not develop any parasitemia upon challenge with WT parasites. In the same immunization protocol, we examined whether the protection against a lethal challenge was dose-dependent. Groups of mice were injected twice at three weeks interval with IL-6 Tg-*Pb*ANKA/LISP2 SPZ at 10³, or 10² SPZ per mouse followed 30 days later by a challenge with 10⁴ WT *Pb*ANKA SPZ (Fig 6A). As shown in Fig. 6B, the two doses of 10³, or 10² of IL-6 Tg-*Pb*ANKA/LISP2 SPZ (group 2 and group 3, respectively) were inefficient in inducing sterile protection as mice developed parasitemia at the same magnitude as the control mice which received WT *Pb*ANKA SPZ. With regard to survival, all mice which received the doses of 10⁴ and 10³ of IL-6 Tg-*Pb*ANKA/LISP2 SPZ survived whereas only 40% of mice which were injected with the dose of 10² IL-6 Tg-*Pb*ANKA/LISP2 SPZ survived (Fig. 6C). In a long-term follow up, all mice which received the dose of 10⁴ IL-6 Tg-*Pb*ANKA/LISP2 SPZ survived whereas those treated with the 10³ and 10² doses of IL-6 Tg-*Pb*ANKA/LISP2 SPZ ultimately died from hyperparasitemia at day 70 and day 72, respectively. These data indicate that the homologous prime boost immunization at the dose of 10⁴ IL-6 Tg-*Pb*ANKA/LISP2 SPZ was found to be optimal in conferring both clinical and sterile immunity.

In order to explore how efficient is the protective immune memory, a similar but slightly different protocol (Fig 9A) was carried out where mice which received the priming and booster injections with IL-6 Tg-*Pb*ANKA/LISP2 SPZ were challenged 60 days after the boost. Similar results were obtained in terms of parasitemia (Group 1, Fig 9B) and survival (Group 1, Fig 9C) as in Fig. 6 with a sterile protection against a challenge with WT *Pb*ANKA SPZ in a homologous prime/boost immunization regimen (Group 1, Fig 9A). More importantly, this sterile protection which occurred 30 or 60 days after the priming with IL-6 Tg-*Pb*ANKA/LISP2 SPZ is indicative of an induction of a robust immune memory. To determine whether clearance of inoculated parasites in protected mice could result in splenic cellularity and macroscopic changes of the spleens, macroscopic examination showed an important splenomegaly at day 6 post-challenge in WT *Pb*ANKA-infected mice (Fig 9D, Group 2, non-protected mice) which was not observed in IL-6 Tg-*Pb*ANKA/LISP2 SPZ protected mice (Fig 9D, Group 1), suggesting a massive leukocyte infiltration in the spleens of WT *Pb*ANKA-infected mice.

### VI.Protection conferred by IL-6 Tg-PbANKA/LISP2 SPZ is dependent on effector CD8⁺ T cells

In order to address whether the protection induced by IL-6 Tg-*Pb*ANKA/LISP2 parasites was dependent on effector CD8+ and/or CD4+ T cells, protected mice which received IL-6 Tg-*Pb*ANKA/LISP2 parasites twice at 3-weeks interval, were treated with normal mouse IgG, anti-CD8 or anti-CD4 depleting antibodies two days before and every other day during a challenge with 10⁴ WT *Pb*ANKA SPZ 30 days after the last boost (Fig. 7A). After challenge, parasite growth and cell depletion efficacy were monitored daily by flow cytometry in blood samples. Efficacy of CD4 and CD8 depletion was continuously monitored during administration of T-cell depleting antibodies (Fig. 11). Interestingly, the measurement of parasitemia indicated a loss of parasite control upon treatment of protected mice with anti-CD8 but not in mice treated with anti-CD4 depleting antibodies (Group 2 and 1, Fig. 7B), in contrast to mice protected but treated with control IgG antibodies (Group 3, Fig. 7B) which did not develop any parasitemia after challenge. In terms of survival, in contrast to mice treated with control IgG and with anti-CD4 depleting antibodies which all survived (Group 3, and 1, Fig. 7C), mice depleted of CD8+ T cells (Groups 2, Fig. 7C), all died from hyperparasitemia. Control naive mice which received WT *Pb*ANKA parasites during challenge (Group 4, Fig. 7B, C) all developed parasitemia and died from cerebral malaria.

To assess how efficient was the anti-malaria protection induced by IL-6 transgenic parasites, protected mice after the first challenge at day 51, were re-challenged twice with WT *Pb*ANKA at day 61 and at day 163 (Group 1, Fig. 6A). As shown in Fig. 6 B, and C, none of the mice of this group developed parasitemia and all mice survived, indicating that protected mice survived and remained parasite free after multiple and temporally distant challenges with WT *Pb*ANKA parasites.

In addition to the elicitation of CD8+ T cells as a predominant defence mechanisms, we examined whether anti-parasite-specific antibodies were induced in mice exposed to the IL-6 transgenic parasites. We quantified anti-SPZ serum IgG antibodies in various immunization protocols as shown in Fig 11A. Overall, the IgG antibody response was found to be induced but at a relatively low level, as the detection threshold was about 1/1000 dilution. It appears that mice which were exposed twice to IL-6 Tg-*Pb*ANKA/LISP2 parasites followed by a challenge with WT *Pb*ANKA parasites (groups 1 and 2, Fig. 11B) developed a significantly higher antibody response as compared to mice which received only WT *Pb*ANKA SPZ or naive mice (groups 1 and 2, respectively, Fig. 11B). It appears also that a challenge with WT *Pb*ANKA SPZ that occurred 60 days after the boost with IL-6 Tg-*Pb*ANKA/LISP2 parasites (group 2) elicited a significantly higher antibody response than that which occurred 30 days after the boost (group 1).

These data demonstrate that the activation of CD8+ but not CD4+ T effector lymphocytes, is indispensable for protection, and that the anti-parasite antibody response may have only a marginal effect.

### VII. Discussion

In this study, the inventors show that *P. berghei* ANKA parasites transfected with the murine IL-6 gene under the control of the *Plasmodium* liver-specific protein 2 (LISP2) promoter, an early marker of liver stage development, express and secrete the mouse IL-6 cytokine. The inventors demonstrated that *Pb*ANKA IL-6 transfectants show, after a brief growth, an abortive development at the liver stage and failed to progress toward blood stage parasites. Furthermore, a homologous prime/boost immunization of mice with IL-6 Tg-*Pb*ANKA/LISP2 SPZ was able to protect mice against multiple and temporally distant challenges with WT *Pb*ANKA parasites, and thus can be considered as a potent anti-malaria vaccine.

The development of effective vaccines against malaria infection and disease was not always successful. One of the most trivial examples is the history of malaria vaccine development where the most established vaccine (RTS,S), a recombinant protein containing regions of the *Plasmodium* CSP protein and targeting the SPZ stage of the parasite confers less than 40% of protection (Chaudhury et al., 2016) (Kaslow and Biernaux, 2015). In an attempt to design effective anti-malaria vaccines, several approaches using transgenic parasites were undertaken. These include the substitution of antigens from other parasite species such as the replacement of the *P. berghei* allele of MSP-1₁₉ with the *P. falciparum* one, (de Koning-Ward et al., 2003), or the replacement of the *csp* gene of rodent parasites by the *csp* gene from *P falciparum* and *P. vivax* (Marin-Mogollon et al., 2018). Another possibility was to express transgenes from other organisms by parasites to enhance their immunogenicity. An attempt was accordingly made with an engineered *Leishmania* ssp to express the monocyte chemoattractant protein (MCP-1) (Conrad et al., 2007) to induce protective responses in susceptible mice.

The inventors have gathered during the last decade several evidence, using various murine models for malaria, that the IL-6 response is critical in controlling the parasite growth by generating an effective anti-parasite immune response (Mathieu et al., 2015); (Demarta-Gatsi et al., 2017; Demarta-Gatsi et al., 2016) (Grand et al., 2020). The inventors decided to focus their study on the IL-6 cytokine for multiple reasons. First, during inflammation, IL-6 was found to be constitutively stored by murine neutrophils (Mathieu et al., 2015) (Terebuh et al., 1992) and its secretion upon TLR ligands is the major inducer of the hepatic acute phase proteins (Gabay and Kushner, 1999). Considering that the liver is a body site where immunological tolerance mechanisms prevail, it was be assumed that this tissue environment could represent an immunologic advantage to pathogens that have elected the liver as the site for their development (Freudenberg et al., 1982) (Lumsden et al., 1988). During the establishment of *Plasmodium* infection, the invasion and development of infectious SPZ inside hepatocytes take advantage of this liver tolerogenic environment. In a recent work, the inventors addressed this issue by using mice deficient in the multidrug resistance-2 gene (Mdr2^{-/-}), which encodes the canalicular phospholipid flippase leading to a complete absence of phospholipids from bile, which were found to spontaneously develop liver injury with a typical inflammatory profil. In this model, the inventors demonstrated that the intra-hepatocyte parasite development was impaired via an IL-6-dependent mechanism (Grand et al., 2020) and this abortive infection resulted in a long-lasting immunity in Mdr2^{-/-} mice against infectious SPZ. Second, IL-6 was selected for its immune-modulatory functions and as a known a key cytokine that supports the growth and enhancement of antibody production by B cells (Hirano et al., 1986), and in support of this, IL-6-deficient mice are impaired in their IgG production upon immunization (Kopf et al., 1994). On the other hand, IL-6 is critical in regulating CD4 T cell differentiation by promoting IL-4 production during T cell activation and IL-21 production, an essential effector cytokine produced by Tfh cells (Barash et al., 2010) (Hirano et al., 1986). Moreover, IL-6 in combination with IL-7 signaling promotes CD8 memory T cell generation after vaccination (Castellino and Germain, 2007). As part of their interest in developing an effective live-vaccine strategy, the inventors postulated that transgenic *Plasmodium* parasites expressing the IL-6 cytokine could have their growth properties impaired and could also provide protection against wild-type parasite challenge. By combining the two main features of the IL-6 cytokine, namely its pro-inflammatory and immunostimulatory properties at the very moment where *Plasmodium* SPZ invade hepatocytes, inducing a hostile environment might provide an impairment for SPZ to progress into their development and the outcome for the infected host might be assessed. These findings prompted the inventors to engineer *Plasmodium* parasites that express the mouse IL-6 gene(with a view to use it in a suitable mouse model for malaria) with the idea that, when inoculated, SPZ would turn on the IL-6 synthesis machinery early during the hepatic phase and the parasite growth would be halted at this stage. Producing *Plasmodium* parasites expressing host cytokines was achieved to determine their capacity to modulate the infection and to characterize the role of the cytokine transgenic parasites to confer resistance to malaria disease. Besides, the use of these IL-6 transgenic parasites for vaccine purposes must meet safety requirements. Accordingly, while developing this concept, the caveat that the inventors imposed was to not allow the parasite to express the IL-6 cytokine systemically, *i.e* during the blood stage. Their strategy was to express the mouse *il-6* gene under the promoter of the liver specific protein 2 (LISP2) selected for its unique profile expression which is limited to the mid-to-late liver stage, as analysis by RT-qPCR showed that LISP2 expression peaked at 48 hpi (hour post infection), and gradually decreased until reaching 60 h post infection (Orito et al., 2013). Subsequently, production of IL-6 by transgenic parasites would be limited at this window of time, not exceeding the hepatic late stage merozoites. To assess whether IL-6 gene transfected *Pb*ANKA parasites do indeed transcribe and secrete the mouse IL-6, the inventors demonstrated that IL-6 was present in supernatants of HepG2 cell line cultured in the presence of IL-6 Tg-*Pb*ANKA/LISP2 SPZ but not in those cultured with WT *Pb*ANKA SPZ, indicating that the IL-6 production machinery was perfectly operational in IL-6 gene transfected *Pb*ANKA parasites. More importantly, *in vivo* analysis showed that at the peak of IL-6 transgenic parasite growth in the liver, namely at 48h post-infection, IL-6 mRNA transcripts were detected at the same time, suggesting an *in vivo* secretion of IL-6. This *in vivo* IL-6 production correlates with the infection pattern of mice infected with IL-6 Tg-*Pb*ANKA/LISP2 SPZ, namely, a complete blockade of the parasite development at the liver stage. This is not related to an intrinsic inability of the IL-6 Tg-*Pb*ANKA/LISP2 SPZ to develop within hepatocyte, and the fact is that IL-6 transgenic parasites develop perfectly well in cultured hepatocytes. Apparently, this was also the case *in vivo* since RT-qPCR analysis indicated a presence of IL-6 Tg-*Pb*ANKA/LISP2 SPZ at 48h post infection, and then they failed to develop further and never gave rise to blood stage. These data are in accordance with the elicitation of an anti-parasite immune response by the IL-6 Tg-*Pb*ANKA/LISP2 SPZ due to the readily transcribed and secreted IL-6 early during parasite development into hepatocytes. This IL-6-driven potent immune response was observed in the MDR2^{-/-} mouse model the inventors reported recently where not only total CD8⁺ and CD4⁺ cells but also CD8⁺ and CD4⁺ tissue resident memory T cells were present at a significantly higher levels in SPZ challenged Mdr2^{-/-} mice (Grand et al., 2020).

To assess whether the inoculation of mice with IL-6 Tg-*Pb*ANKA/LISP2 SPZ was able to prime the immune system to cope with a subsequent challenge with WT *Pb*ANKA SPZ, the inventors designed several protocols exploring various parameters including single multi-dose priming, and homologous prime/boost immunization regimens with various time intervals between the immunization and the challenge with WT *Pb*ANKA SPZ which was spanning between 30 and 60 days. Their results indicate that a single dose of IL-6 Tg-*Pb*ANKA/LISP2 SPZ, although it protected mice from developing cerebral malaria symptoms following challenge as compared to WT *Pb*ANKA infection, did not fully protect mice against blood stage infection with, however, a significantly prolonged pre-patent period. In contrast, a full anti-disease and anti-parasite immunity were achieved after a homologous prime/boost delivery of IL-6 Tg-*Pb*ANKA/LISP2 SPZ whether the challenge with WT parasites occurred at 30 days or even at 60 days after the booster dose of IL-6 Tg-*Pb*ANKA/LISP2 SPZ. Furthermore, this protection was persistent and was maintained even after multiple challenges with WT parasites. This is indicative of a long-lasting immune memory elicited by IL-6 transgenic parasites.

The failure to mount robust CD4⁺ and CD8⁺ T cell responses during natural infection via infectious mosquito bites is believed to be related to a relatively low amounts of inoculated SPZ (about 100 SPZ), ultimately leading to a very limited number of infected hepatocytes, followed by a rapid release of merozoites in the bloodstream (2 days) at least in rodents. To overcome this insufficient parasite antigen availability during the short window of liver stage development, a more optimal liver stage antigen-specific CD4⁺ and CD8⁺ T cell responses can be triggered by a more persistent liver stage-arresting genetically attenuated parasites (GAP). In support of this, efficient CD8⁺ T cell responses can be elicited by late liver stage-arresting GAPs which provide a persistent source of parasite antigens (Goswami et al., 2019). *If* CD8⁺ T cells are critical effectors for the control of *Plasmodium-infected* hepatocytes in various species including humans, non-human primates and rodents (Kelemen et al., 2019), CD4⁺ T cells that recognize parasite-derived immunogenic peptides in the context of MHC class II molecules expressed on infected hepatocytes also contribute in effector mechanisms against *Plasmodium* parasite liver stages (Rénia et al., 1993). CD4⁺ T cells were also found to participate to protection against *Plasmodium* infection by inducing antibody production and macrophage activation (Langhorne et al., 2008) (Jafarshad et al., 2007). As an alternative strategy, we generated a liver stage-arresting IL-6 transgenic *Plasmodium* parasites which not only failed to produce blood stage parasites but also conferred a long-lasting protection against virulent challenge with WT parasites. The requirement of only CD8 T cells in the protection induced by IL-6 transgenic parasites was demonstrated by the lack of the control of parasite development into blood stage in mice treated with anti-CD8 but not in those treated with anti-CD4 depleting antibodies.

In contrast to our IL-6 transgenic parasites which confer an efficient and long-lasting protection against parasite growth and against disease, other investigators have attempted to produce transgenic "suicide" parasite organisms, such as *Leishmania,* with more or less success. This was the case of transgenic *L. major* which was stably transfected with the mouse IFN-γ and found to secrete this cytokine but failed to protect susceptible BALB/c mice from infection (Tobin et al., 1993), or transgenic *L. major* secreting the mouse granulocyte-macrophage colony stimulating factor (GM-CSF) which were more promising but were found to confer only a partial protective response since infection *in vivo* showed only a delayed development of lesions in susceptible mice (Dumas et al., 2003). With regard to *Plasmodium* parasite, only one old study demonstrated that *P. knowlesi* parasites, transfected with the IFN-γ gene from rhesus monkey under control of the heterologous *P. berghei* apical membrane antigen 1 promoter, express bioactive IFN-y.The authors did not however investigate further the infectious behavior and whether this transgenic parasite was protective or not against an infectious parasite challenge (Ozwara et al., 2003). In contrast to the present study, none of these reports could investigate the mechanisms underlying these partial protective capabilities.

In conclusion, the inventors engineered IL-6 transgenic *Plasmodium berghei* parasites suitable for use in a relevant mouse model of malaria and demonstrated that they were able to synthesize and secrete the murine IL-6 cytokine. Immunization with these IL-6 transgenic parasites abolished parasite growth at the PEE stage and established a long-lasting immune protection against a lethal parasite challenge. Inventors' data indicate that expression of the murine IL-6 gene under LISP2 promoter which restricts its production to the liver, opens new perspectives for the development of an effective vaccine against exoerythrocytic forms of *Plasmodium* parasite.

### Bibliography

Augustijn, K.D., R. Kleemann, J. Thompson, T. Kooistra, C.E. Crawford, S.E. Reece, A. Pain, A.H. Siebum, C.J. Janse, and A.P. Waters. 2007. Functional characterization of the Plasmodium falciparum and P. berghei homologues of macrophage migration inhibitory factor. Infection and immunity 75:1116-1128.

Barash, H., R.G. E, Y. Edrei, E. Ella, A. Israel, I. Cohen, N. Corchia, T. Ben-Moshe, O. Pappo, E. Pikarsky, D. Goldenberg, Y. Shiloh, E. Galun, and R. Abramovitch. 2010. Accelerated carcinogenesis following liver regeneration is associated with chronic inflammation-induced double-strand DNA breaks. Proceedings of the National Academy of Sciences of the United States of America 107:2207-2212.

Barash S. et al 2002 Human secretory signal peptide description by hidden Markov model and generation of a strong artificial signal peptide for secreted protein expression. Biochemical and Biophysical Research Communications 294 (2002) 835-842 - https://www.sciencedirect.com/science/article/abs/pii/S0006291X02005661?via%3Dihub

Birnbaum, J., S. Flemming, N. Reichard, A.B. Soares, P. Mesén-Ramírez, E. Jonscher, B. Bergmann, and T. Spielmann. 2017. A genetic system to study Plasmodium falciparum protein function. Nature methods 14:450-456.

Castellino, F., and R.N. Germain. 2007. Chemokine-guided CD4+ T cell help enhances generation of IL-6RalphahighIL-7Ralpha high prememory CD8+ T cells. Journal of immunology (Baltimore, Md. : 1950) 178:778-787.

Chamekh, M., V. Vercruysse, M. Habib, M. Lorent, M. Goldman, A. Allaoui, and B. Vray. 2005. Transfection of Trypanosoma cruzi with host CD40 ligand results in improved control of parasite infection. Infection and immunity 73:6552-6561.

Chaudhury, S., C.F. Ockenhouse, J.A. Regules, S. Dutta, A. Wallqvist, E. Jongert, N.C. Waters, F. Lemiale, and E. Bergmann-Leitner. 2016. The biological function of antibodies induced by the RTS,S/AS01 malaria vaccine candidate is determined by their fine specificity. Malaria journal 15:301.

Conrad, S.M., D. Strauss-Ayali, A.E. Field, M. Mack, and D.M. Mosser. 2007. Leishmania-derived murine monocyte chemoattractant protein 1 enhances the recruitment of a restrictive population of CC chemokine receptor 2-positive macrophages. Infection and immunity 75:653-665.

de Koning-Ward, T.F., R.A. O'Donnell, D.R. Drew, R. Thomson, T.P. Speed, and B.S. Crabb. 2003. A new rodent model to assess blood stage immunity to the Plasmodium falciparum antigen merozoite surface protein 119 reveals a protective role for invasion inhibitory antibodies. The Journal of experimental medicine 198:869-875.

Demarta-Gatsi, C., R. Peronet, L. Smith, S. Thiberge, R. Ménard, and S. Mécheri. 2017. Immunological memory to blood-stage malaria infection is controlled by the histamine releasing factor (HRF) of the parasite. Scientific reports 7:9129.

Demarta-Gatsi, C., L. Smith, S. Thiberge, R. Peronet, P.H. Commere, M. Matondo, L. Apetoh, P. Bruhns, R. Ménard, and S. Mécheri. 2016. Protection against malaria in mice is induced by blood stage-arresting histamine-releasing factor (HRF)-deficient parasites. The Journal of experimental medicine 213: 1419-1428.

Dumas, C., A. Muyombwe, G. Roy, C. Matte, M. Ouellette, M. Olivier, and B. Papadopoulou. 2003. Recombinant Leishmania major secreting biologically active granulocyte-macrophage colony-stimulating factor survives poorly in macrophages in vitro and delays disease development in mice. Infection and immunity 71:6499-6509.

Field, A.E., S. Wagage, S.M. Conrad, and D.M. Mosser. 2007. Reduced pathology following infection with transgenic Leishmania major expressing murine CD40 ligand. Infection and immunity 75:3140-3149.

Freudenberg, M.A., N. Freudenberg, and C. Galanos. 1982. Time course of cellular distribution of endotoxin in liver, lungs and kidneys of rats. British journal of experimental pathology 63:56-65.

Gabay, C., and I. Kushner. 1999. Acute-phase proteins and other systemic responses to inflammation. The New Englandjournal of medicine 340:448-454.

Goswami, D., N.K. Minkah, and S.H.I. Kappe. 2019. Designer Parasites: Genetically Engineered Plasmodium as Vaccines To Prevent Malaria Infection. Journal of immunology (Baltimore, Md. : 1950) 202:20-28.

Grand, M., M. Waqasi, C. Demarta-Gatsi, Y. Wei, R. Peronet, P.-H. Commere, A. Puig, J. Axelrod, R. Caldelari, V. Heussler, R. Amino, and S. Mecheri. 2020. Hepatic inflammation confers protective immunity against liver stages of malaria parasite. Front Immunol 11:1-17.

Haussig, J.M., K. Matuschewski, and T.W. Kooij. 2011. Inactivation of a Plasmodium apicoplast protein attenuates formation of liver merozoites. Molecular microbiology 81:1511-1525.

Hirano, T., K. Yasukawa, H. Harada, T. Taga, Y. Watanabe, T. Matsuda, S. Kashiwamura, K. Nakajima, K. Koyama, A. Iwamatsu, and et al. 1986. Complementary DNA for a novel human interleukin (BSF-2) that induces B lymphocytes to produce immunoglobulin. Nature 324:73-76.

Jafarshad, A., M.H. Dziegiel, R. Lundquist, L.K. Nielsen, S. Singh, and P.L. Druilhe. 2007. A novel antibody-dependent cellular cytotoxicity mechanism involved in defense against malaria requires costimulation of monocytes FcgammaRII and FcgammaRIII. Journal of immunology (Baltimore, Md. : 1950) 178:3099-3106.

Janse, C.J., J. Ramesar, and A.P. Waters. 2006. High-efficiency transfection and drug selection of genetically transformed blood stages of the rodent malaria parasite Plasmodium berghei. Nature protocols 1:346-356.

Kaslow, D.C., and S. Biernaux. 2015. RTS,S: Toward a first landmark on the Malaria Vaccine Technology Roadmap. Vaccine 33:7425-7432.

Kelemen, R.K., H. Rajakaruna, I.A. Cockburn, and V.V. Ganusov. 2019. Clustering of Activated CD8 T Cells Around Malaria-Infected Hepatocytes Is Rapid and Is Driven by Antigen-Specific Cells. Front Immunol 10:2153.

Kopf, M., H. Baumann, G. Freer, M. Freudenberg, M. Lamers, T. Kishimoto, R. Zinkernagel, H. Bluethmann, and G. Kohler. 1994. Impaired immune and acute-phase responses in interleukin-6-deficient mice. Nature 368:339-342.

Kublin, J.G., S.A. Mikolajczak, B.K. Sack, M.E. Fishbaugher, A. Seilie, L. Shelton, T. VonGoedert, M. Firat, S. Magee, E. Fritzen, W. Betz, H.S. Kain, D.A. Dankwa, R.W. Steel, A.M. Vaughan, D. Noah Sather, S.C. Murphy, and S.H. Kappe. 2017. Complete attenuation of genetically engineered Plasmodium falciparum sporozoites in human subjects. Science translational medicine 9*:*

Langhorne, J., F.M. Ndungu, A.M. Sponaas, and K. Marsh. 2008. Immunity to malaria: more questions than answers. Nature immunology 9:725-732.

Lumsden, A.B., J.M. Henderson, and M.H. Kutner. 1988. Endotoxin levels measured by a chromogenic assay in portal, hepatic and peripheral venous blood in patients with cirrhosis. Hepatology (Baltimore, Md.) 8:232-236.

Manzoni, G., S. Briquet, V. Risco-Castillo, C. Gaultier, S. Topçu, M.L. Iv 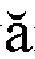 nescu, J.F. Franetich, B. Hoareau-Coudert, D. Mazier, and O. Silvie. 2014. A rapid and robust selection procedure for generating drug-selectable marker-free recombinant malaria parasites. *Scientific reports* 4:4760.

Marin-Mogollon, C., F.J.A. van Pul, S. Miyazaki, T. Imai, J. Ramesar, A.M. Salman, B.M.F. Winkel, A.S. Othman, H. Kroeze, S. Chevalley-Maurel, A. Reyes-Sandoval, M. Roestenberg, B. Franke-Fayard, C.J. Janse, and S.M. Khan. 2018. Chimeric Plasmodium falciparum parasites expressing Plasmodium vivax circumsporozoite protein fail to produce salivary gland sporozoites. Malaria journal 17:288.

Mathieu, C., C. Demarta-Gatsi, A. Porcherie, S. Brega, S. Thiberge, K. Ronce, L. Smith, R. Peronet, R. Amino, R. Ménard, and S. Mécheri. 2015. Plasmodium berghei histamine-releasing factor favours liver-stage development via inhibition of IL-6 production and associates with a severe outcome of disease. Cellular microbiology 17:542-558.

Miller, J.L., A. Harupa, S.H. Kappe, and S.A. Mikolajczak. 2012. Plasmodium yoelii macrophage migration inhibitory factor is necessary for efficient liver-stage development. Infection and immunity 80:1399-1407.

Nganou-Makamdop, K., and R.W. Sauerwein. 2013. Liver or blood-stage arrest during malaria sporozoite immunization: the later the better? Trends in parasitology 29:304-310.

Nkrumah LJ, Muhle RA, Moura PA, Ghosh P, Hatfull GF, Jacobs WR Jr, Fidock DA. 2006 Efficient site-specific integration in Plasmodium falciparum chromosomes mediated by mycobacteriophage Bxb1 integrase. Nat Methods. 2006 Aug;3(8):615-21. doi: 10.1038/nmeth904. Erratum in: Nat Methods. 2006 Sep;3(9):763. PMID: 16862136; PMCID: PMC2943413.

Orito, Y., T. Ishino, S. Iwanaga, I. Kaneko, T. Kato, R. Menard, Y. Chinzei, and M. Yuda. 2013. Liver-specific protein 2: a Plasmodium protein exported to the hepatocyte cytoplasm and required for merozoite formation. Molecular microbiology 87:66-79.

Ozwara, H., J.A. Langermans, C.H. Kocken, A. van der Wel, P.H. van der Meide, R.A. Vervenne, J.M. Mwenda, and A.W. Thomas. 2003. Transfected Plasmodium knowlesi produces bioactive host gamma interferon: a new perspective for modulating immune responses to malaria parasites. Infection and immunity 71:4375-4381.

Rénia, L., D. Grillot, M. Marussig, G. Corradin, F. Miltgen, P.H. Lambert, D. Mazier, and G. Del Giudice. 1993. Effector functions of circumsporozoite peptide-primed CD4+ T cell clones against Plasmodium yoelii liver stages. Journal of immunology (Baltimore, Md. : 1950) 150:1471-1478.

Roestenberg M. et al. 2020 A double-blind, placebo-controlled phase 1/2a trial of the genetically attenuated malaria vaccine PfSPZ-GA1. Sci. Transl. Med. 12, eaaz5629 (2020) 1-9

Sun, T., T. Holowka, Y. Song, S. Zierow, L. Leng, Y. Chen, H. Xiong, J. Griffith, M. Nouraie, P.E. Thuma, E. Lolis, C.J. Janse, V.R. Gordeuk, K. Augustijn, and R. Bucala. 2012. A Plasmodium-encoded cytokine suppresses T-cell immunity during malaria. Proceedings of the National Academy of Sciences of the United States of America 109:E2117-2126.

Terebuh, P.D., I.G. Otterness, R.M. Strieter, P.M. Lincoln, J.M. Danforth, S.L. Kunkel, and S.W. Chensue. 1992. Biologic and immunohistochemical analysis of interleukin-6 expression in vivo. Constitutive and induced expression in murine polymorphonuclear and mononuclear phagocytes. The American journal of pathology 140:649-657.

Tobin, J.F., S.L. Reiner, F. Hatam, S. Zheng, C.L. Leptak, D.F. Wirth, and R.M. Locksley. 1993. Transfected Leishmania expressing biologically active IFN-gamma. Journal of immunology (Baltimore, Md. : 1950) 150:5059-5069

## Claims

1. A transgene construct which comprises a polynucleotide encoding a mammalian IL-6 protein, in particular a human IL-6 protein, wherein the polynucleotide is under the control of a transcription and expression control nucleic acid comprising a promoter of a gene of a *Plasmodium* parasite wherein this gene is selected among genes expressed during exoerythrocytic stage of *Plasmodium* parasite life cycle in a mammalian host, and wherein the transgene comprises a nucleic acid encoding a signal peptide enabling secretion of the expressed IL-6 protein.

2. The transgene construct of claim 1 which comprises the following nucleic acid sequences from 5' to 3' in the polynucleotide:
- a nucleotide sequence of the promoter of a gene of a *Plasmodium* parasite,
- a nucleotide sequence encoding the signal peptide, in particular a signal peptide of a gene of a *Plasmodium* parasite,
- a nucleotide sequence encoding the IL-6 protein,
- optionally sequence(s) of restriction site(s) suitable for cloning of the above sequences in the transgene and/or for insertion of the transgene construct in the *Plasmodium* parasite genome restriction sites suitable for insertion of the transgene construct in the *Plasmodium* parasite genome,
wherein the nucleic acid sequences are functionally associated within the transgene to enable expression of the IL-6 protein from a *Plasmodium* parasite genetically modified with the transgene.

3. The transgene construct of claim 1 or 2 wherein the transcription and expression control nucleic acid comprises a promoter of a *Plasmodium* gene which is expressed in the hepatic stage of the *Plasmodium* parasite life cycle, such as a gene expressed in exoerythrocytic forms (EEF), in particular in late EEFs.

4. The transgene construct of any one of claims 1 to 3, wherein the polynucleotide encoding the mammalian IL-6 protein comprises a nucleotide sequence encompassing the Open Reading Frame (ORF) of the IL-6 gene and is selected in the group of : SEQ ID NO. 1, SEQ ID NO. 2, SEQ ID NO. 3, SEQ ID NO. 4.

5. The transgene construct of any one claims 1 to 4, wherein the promoter of the gene of a *Plasmodium* parasite is the promoter of a gene selected in the group of the LISP2 gene of *P. Berghei* (SEQ ID No. 10), when the ORF encodes murine IL-6, or of LISP2 gene of *P falciparum* (SEQ ID No. 11), or of LISP2 gene of *P.vivax,* when the ORF encodes human IL-6.

6. The transgene construct of any one of claims 1 to 5, wherein the promoter of the gene of a *Plasmodium* parasite is the promoter of a gene selected in the group of the *LISP1 or LISP2* gene of *P. Berghei* when the ORF encodes murine IL-6 or LISP-1 gene of *P. falciparum* (SEQ ID No. 12), *P. vivax, P. malariae or P. ovale* or *P. knowlesi* when the ORF encodes human IL-6, preferably *P. falciparum.*

7. The transgene construct of any one of claims 1 to 6, wherein the nucleic acid encoding a signal peptide for secretion of the expressed IL-6 protein is selected from the group of : the nucleotide sequence (SEQ ID No. 5) encoding the signal peptide of *P. berghei* LISP2, the nucleotide sequence encoding the signal peptide of *P. berghei* LISP1, the nucleotide sequence (SEQ ID No. 8) encoding the signal peptide of the human albumin, and the nucleotide sequence (SEQ ID No. 6) encoding the peptide signal of SEQ ID No. 7.

8. The transgene according to any one of claims 1 to 7, wherein the polynucleotide comprises from 5' to 3':
a. a nucleotide sequence of the promoter of a gene of a *Plasmodium* parasite, selected from the group of SEQ ID NO. 10, SEQ ID NO. 11 (both promoters of the LISP2 gene) and SEQ ID NO. 12 (promoter of PfLISP1 gene),
b. a nucleotide sequence encoding a signal peptide, in particular a nucleotide sequence of SEQ ID NO. 5 encoding a signal peptide of a LISP2 gene of a *Plasmodium Berghei* parasite, a nucleotide sequence of SEQ ID NO. 6, or a nucleotide sequence of SEQ ID NO. 8,
c. a nucleotide sequence encoding the IL-6 protein, selected from the group of SEQ ID NO. 1, SEQ ID NO. 2, SEQ ID NO. 3 and SEQ ID No. 4,
d. optionally nucleotide sequence(s) of restriction site(s) suitable for cloning of sequences a., b. and c., in the transgene and/or for insertion of the transgene construct in the *Plasmodium* parasite genome.

9. An expression plasmid vector recombined with the transgene construct of any one of claims 1 to 8.

10. A transgenic *Plasmodium* parasite wherein the transgene encodes a mammalian IL-6 protein, in particular a human IL-6 protein, and is a transgene construct of any one of claims 1 to 8.

11. The transgenic *Plasmodium* parasite according to claim 10, wherein the transgene comprises a nucleotide sequence selected in the group of : SEQ ID NO. 1, SEQ ID NO. 2, SEQ ID NO. 3 and SEQ ID NO. 4, in particular wherein the transgene comprises the sequence of SEQ ID NO. 15.

12. The transgenic *Plasmodium* parasite of any one of claims 10 or 11 wherein the transgene is integrated in the *Plasmodium* genome.

13. The transgenic *Plasmodium* parasite of any one of claims 10 to 12 which is a live attenuated *Plasmodium* parasite capable of infecting human, in particular a genetically attenuated parasite (GAP).

14. The transgenic *Plasmodium* parasite of any one of claims 10 to 14 which is a transgenic parasite of *Plasmodium falciparum, Plasmodium vivax, Plasmodium malariae, Plasmodium knowlesi* or *Plasmodium ovale.*

15. The transgenic *Plasmodium* parasite of any one of claims 10 to 14 which is a preerythrocytic live attenuated *Plasmodium* parasite capable of infecting human, in particular an early exoerythrocytic form (EEF), in particular is a sporozoite selected in the group of infectious sporozoites, genetically attenuated sporozoites or radiation attenuated sporozoites.

16. A composition suitable for inducing, promoting or enhancing resistance against *Plasmodium* parasite infection in a mammalian host wherein the composition comprises a transgenic *Plasmodium* parasite capable of infecting a human host, in particular a transgenic parasite of *Plasmodium falciparum* or *Plasmodium vivax* species, wherein the transgenic parasite is a transgenic parasite of any one of claims 10 to 15, preferably wherein the composition is devoid of an exogenous adjuvant of the immune response.

17. The transgenic *Plasmodium* parasite of any one of claims 10 to 15 or the composition according to claim 16 for use in promoting or eliciting resistance against *Plasmodium* parasite infection in a mammalian host, in particular a human host.

18. The transgenic *Plasmodium* parasite of any one of claims 10 to 15 or the composition according to claim 16 for use in eliciting or boosting innate immune and/or adaptive immune response against *Plasmodium* liver-stage infection or *Plasmodium* development in a mammalian host, in particular a human host.

19. The transgenic *Plasmodium* parasite of any one of claims 10 to 15 or the composition according to claim 16 for use in preventive protection against malaria disease in a mammalian especially human host, in particular by conferring sterile protection to the mammalian host, in particular human host.

20. The transgenic *Plasmodium* parasite of any one of claims 10 to 15 or the composition according to claim 16 for use according to any one of claims 17 to 19 wherein the dose(s) of administered transgenic parasite is from 10⁵ to 10⁶ sporozoites, in particular 1.35x10⁵ to 9.0x10⁵ sprorozoites in a human host.

21. The transgenic *Plasmodium* parasite of any one of claims 10 to 15 or the composition according to claim 16 for use according to any one of claims 17 to 20 wherein the administration regimen comprises administration of at least two doses of the transgenic *Plasmodium* parasite or of the composition and/or comprises a prime-boost regimen.
